(19)

Europäisches Patentamt
European Patent Office
Office européen des brevets

(11)  **EP 4 403 162 A1**

(12)  **EUROPEAN PATENT APPLICATION**
published in accordance with Art. 153(4) EPC

(43) Date of publication:
24.07.2024  Bulletin 2024/30

(21) Application number: 22870061.3

(22) Date of filing: 16.09.2022

(51) International Patent Classification (IPC):
*A61K 6/887* (2020.01)          *A61K 6/813* (2020.01)
*A61K 6/816* (2020.01)

(52) Cooperative Patent Classification (CPC):
**A61K 6/813; A61K 6/816; A61K 6/887**

(86) International application number:
**PCT/JP2022/034835**

(87) International publication number:
**WO 2023/042915 (23.03.2023 Gazette 2023/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  17.09.2021  JP 2021152632

(71) Applicant: **Kuraray Noritake Dental Inc.
Kurashiki-shi, Okayama 710-0801 (JP)**

(72) Inventor: **SUZUKI, Kenji
Tainai-shi, Niigata 959-2653 (JP)**

(74) Representative: **D Young & Co LLP
3 Noble Street
London EC2V 7BQ (GB)**

(54)  **STEREOLITHOGRAPHY PLATE DENTURE MANUFACTURING KIT, AND METHOD FOR MANUFACTURING PLATE DENTURE**

(57)    The present invention relates to a plate denture production kit for stereolithography including a curable composition for a denture base (I) containing a (meth)acrylic based polymerizable monomer component (A) containing a (meth)acrylic based polymerizable monomer (m) as a major monomer, and a photopolymerization initiator, and a composition for a prosthetic tooth (II) containing a (meth)acrylic based polymerizable monomer component (B) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (B), and a method of producing a plate denture.

**EP 4 403 162 A1**

**Description**

Technical Field

The present invention relates to a plate denture production kit for stereolithography including a curable composition for a denture base for stereolithography and a composition for a prosthetic tooth, and a method of producing a plate denture including adhering a particular denture base and a particular prosthetic tooth by using a particular adhesive composition.

Background Art

Dentures have been produced by the lost-wax process shown below. First, a mold of the oral cavity of the patient is prepared, a base plate is formed on a gypsum model produced based on the mold, and a gingival part formed of wax, which is referred to as a wax rim, is further built thereon to produce a biteplate. Next, after bite taking, prosthetic teeth formed of an acrylic resin are arranged, and a wax denture is completed through occlusal adjustment and gingival formation. Subsequently, after performing an operation referred to as embedding, i.e., embedding the wax denture and the gypsum model in a flame, which is referred to as a flask, with gypsum, the wax is melted and removed with hot water, the plate is also removed, and then an acrylic resin is poured into the cavity and then cured, followed by performing an operation for finishing, thereby completing the denture. This method not only includes a large number of process steps, but also requires extremely high dimensional accuracy on the order of several micrometers for the denture production, requiring not only a large amount of time but also the skill of the dental mechanic.
In recent years, CAD/CAM systems that designs dentures on a screen using a computer and produces dentures by cutting from a block material formed of a resin or ceramics are attracting attention as a method capable of stably providing dentures of a certain quality within a short period of time, and the denture designing and production systems, such as Cerec System, have become widespread. Among these, the system using a stereolithographic 3D printer is rapidly spreading.
In the stereolithographic method, in general, it is necessary to adhere a base produced by stereolithography and a prosthetic tooth. Examples of the prosthetic tooth include a PMMA prosthetic tooth produced through injection molding, a hard resin prosthetic tooth produced through cutting, and a prosthetic tooth produced by molding of a photocurable resin composition that is different from the base. Most of the adhesives are a powder-liquid type resin constituted by methyl methacrylate (MMA) having a polymerization initiator mixed therein and acrylic resin powder. In the lost-wax method, a denture excellent in strength and toughness can be easily obtained since the base, the prosthetic tooth, and the adhesive are basically constituted by the same kind of materials, resulting in firm adhesion between the base and the prosthetic tooth due to the small property difference and the good affinity between them. In the stereolithographic method, however, a polymerizable compound having particularly high curability is necessarily used since the compound is instantaneously cured with light, and therefore a crosslinkable monomer and a monomer exhibiting high cohesive force are used, which results in a problem of embrittlement. Furthermore, the adhesive is difficult to penetrate thereto, and thereby not only a sufficient adhesion force cannot be obtained, but also the base and the prosthetic tooth are difficult to unite with each other due to the property difference between them, which results in a problem that the denture cannot have the sufficient strength and toughness.
In view of the background described above, PTL 1 describes a production example of a denture obtained by adhering a denture base produced by using a CAD/CAM system and a prosthetic tooth formed of an acrylic resin with a powder-liquid type resin containing MMA and PMMA. PTL 2 describes an example of a denture base material for stereolithography excellent in strength and toughness.

Citation List

Patent Literatures

   PTL 1: WO2018/159507
   PTL 2: WO2018/181832

Summary of Invention

Technical Problem

However, PTLs 1 and 2 fail to describe the strength and toughness in the state where the denture base and the prosthetic tooth are adhered to each other.

Under the circumstances, an object of the present invention is to provide a denture that is excellent in strength and toughness for a combination of a denture base produced by stereolithography and a prosthetic tooth.

Solution to Problem

The present invention encompasses the following inventions.

[1] A plate denture production kit for stereolithography including

a curable composition for a denture base (I) containing a (meth)acrylic based polymerizable monomer component (A) containing a (meth)acrylic based polymerizable monomer (m) as a major monomer, and a photopolymerization initiator, and

a composition for a prosthetic tooth (II) containing a (meth)acrylic based polymerizable monomer component (B) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (B).

[2] The plate denture production kit for stereolithography according to the item [1], in which the (meth)acrylic based polymerizable monomer (m) is a polyfunctional (meth)acrylic based polymerizable monomer (a-1).

[3] The plate denture production kit for stereolithography according to the item [2], in which the polyfunctional (meth)acrylic based polymerizable monomer (a-1) is at least one kind selected from a urethanated (meth)acrylate ester compound and an aromatic compound based (meth)acrylate ester compound having no urethane bond.

[4] The plate denture production kit for stereolithography according to any one of the items [1] to [3], in which the (meth)acrylic based polymerizable monomer component (A) contains a monofunctional (meth)acrylic based polymerizable monomer (a-2).

[5] The plate denture production kit for stereolithography according to any one of the items [1] to [4], in which the (meth)acrylic based polymerizable monomer component (B) contains a monofunctional (meth)acrylic based polymerizable monomer (a-2).

[6] The plate denture production kit for stereolithography according to the item [4] or [5], in which the monofunctional (meth)acrylic based polymerizable monomer (a-2) is a (meth)acrylate ester based polymerizable monomer having an aromatic ring.

[7] The plate denture production kit for stereolithography according to any one of the items [1] to [6], in which the curable composition for a denture base (I) has a content of the (meth)acrylic based polymerizable monomer (m) of 50% by mass or more based on 100% by mass of the (meth)acrylic based polymerizable monomer component (A).

[8] The plate denture production kit for stereolithography according to any one of the items [1] to [7], in which the composition for a prosthetic tooth (II) has a content of the (meth)acrylic based polymerizable monomer (m) of 50% by mass or more based on 100% by mass of the (meth)acrylic based polymerizable monomer component (B).

[9] The plate denture production kit for stereolithography according to any one of the items [1] to [8], in which the curable composition for a denture base (I) contains the (meth)acrylic based polymerizable monomer component (A) in an amount of 40% by mass or more based on 100% by mass of the curable composition for a denture base (I).

[10] The plate denture production kit for stereolithography according to any one of the items [1] to [9], in which the composition for a prosthetic tooth (II) contains the (meth)acrylic based polymerizable monomer component (B) in an amount of 40% by mass or more based on 100% by mass of the composition for a prosthetic tooth (II).

[11] The plate denture production kit for stereolithography according to any one of the items [1] to [10], in which the plate denture production kit for stereolithography includes

the curable composition for a denture base (I),

the composition for a prosthetic tooth (II), and

an adhesive composition containing a composition (III) containing a (meth)acrylic based polymerizable monomer component (C) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (C).

[12] A method of producing a plate denture, including adhering

a denture base containing a cured product of a curable composition for a denture base (I) containing a (meth)acrylic based polymerizable monomer component (A) containing a (meth)acrylic based polymerizable monomer (m) as a major monomer, and a photopolymerization initiator, and

a prosthetic tooth containing a cured product of a composition for a prosthetic tooth (II) containing a (meth)acrylic based polymerizable monomer component (B) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (B),

with an adhesive composition containing a composition (III) containing a (meth)acrylic based polymerizable monomer component (C) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (C).

Advantageous Effects of Invention

The present invention can provide a denture that is excellent in strength and toughness for a combination of a denture base produced by stereolithography and a prosthetic tooth.

Description of Embodiments

The present invention will be described in detail with reference to embodiments below.

In the description herein, the upper limit values and the lower limit values of the numeral ranges (such as the contents of the components, the values calculated from the components, and the property values) can be appropriately combined.

Therefore, in the description herein, the lower limit values and the upper limit values described in a stepwise manner for the numeral ranges of the same item can be independently combined. For example, based on the description "preferably 10 to 90, and more preferably 30 to 60" for the same item, the "preferred lower limit value (10)" and the "more preferred upper limit value (60)" can be combined to make a range of "10 to 60".

As for the numeral ranges, for example, based on the description "preferably 10 to 90, and more preferably 30 to 60", only the lower limit value can be defined as "10 or more" or "30 or more" without particularly limiting the upper limit value. Similarly, only the upper limit value can be defined as "90 or less" or "60 or less" without particularly limiting the lower limit value.

Unless otherwise indicated, the description "10 to 90" simply for the numeral range means a range of 10 or more and 90 or less.

As similar to the above, for example, based on the description "preferably 10 or more, and more preferably 30 or more" and the description "preferably 90 or less, and more preferably 60 or less" for the same item, the "preferred lower limit value (10)" and the "more preferred upper limit value (60)" can be combined to make a range of "10 or more and 60 or less". As similar to the above, only the lower limit value can be defined as " 10 or more" or "30 or more", and similarly, only the upper limit value can be defined as "90 or less" or "60 or less".

In the description herein, the expression "(meth)acrylic" is used as the concept encompassing both "methacrylic" or "acrylic". This is similarly applied to the analogous expressions including "(meth)acrylate", "(meth)acrylamide", "(meth)acryloyloxy", and the like.

In the description herein, unless otherwise indicated, the terms having the same symbol (for example, the "composition (I)", the "composition (II)", the "composition (III)", the "monomer (m)", the "monomer (o)", the "monomer (a-1)", the "monomer (a-2)", the "component (A)", the "component (B)", and the "component (C)") indicate the same item.

In the description herein, unless otherwise indicated, the descriptions of "strength" and the "toughness" mean the descriptions of "strength" and the "toughness" respectively of the prosthetic tooth produced from the plate denture production kit for stereolithography as one embodiment of the present invention.

In the description herein, unless otherwise indicated, the "denture" means a denture that is produced from a plate denture production kit for stereolithography that is one embodiment of the present invention.

[Plate Denture Production Kit for Stereolithography]

A plate denture production kit for stereolithography that is one embodiment of the present invention includes a curable composition for a denture base (I) containing a (meth)acrylic based polymerizable monomer component (A) containing a (meth)acrylic based polymerizable monomer (m) as a major monomer, and a photopolymerization initiator, and a composition for a prosthetic tooth (II) containing a (meth)acrylic based polymerizable monomer component (B) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (B).

[Curable Composition for Denture Base (I)]

The curable composition for a denture base (I) (which may be hereinafter referred simply to as a "composition (I)") contains a (meth)acrylic based polymerizable monomer component (A) (which may be hereinafter referred simply to as a "component (A)") containing a (meth)acrylic based polymerizable monomer (m) (which may be hereinafter referred simply to as a "monomer (m)") as a major monomer, and a photopolymerization initiator.

<(Meth)acrylic based Polymerizable Monomer Component (A)>

The (meth)acrylic based polymerizable monomer component (A) is a component that is constituted by one kind of the (meth)acrylic based polymerizable monomer alone, or by two or more kinds thereof, and contains a (meth)acrylic based polymerizable monomer (m) as a major monomer.
In the description herein, the "major monomer" means the (meth)acrylic based polymerizable monomer that has the largest content among the (meth)acrylic based polymerizable monomers constituting the component (A).
In the description herein, the "(meth)acrylic based polymerizable monomer component" means a component that is constituted by one kind of the (meth)acrylic based polymerizable monomer alone, or by two or more kinds thereof, which is applied to the component (A), and similarly to the "(meth)acrylic based polymerizable monomer component (B)" and the "(meth)acrylic based polymerizable monomer component (C)" described later.
In the description herein, the "(meth)acrylic based polymerizable monomer" means one specific compound constituting the "(meth)acrylic based polymerizable monomer component", i.e., a particular (meth)acrylic based polymerizable monomer, which is applied to the monomer (m), and similarly to the "polyfunctional (meth)acrylic based polymerizable monomer (a-1)" and the "monofunctional (meth)acrylic based polymerizable monomer (a-2)" described later.
The content of the monomer (m) contained as the major monomer in the component (A) is the largest in the component (A), is not particularly limited, as far as being the content of a (meth)acrylic based polymerizable monomer (o) (which may be hereinafter referred simply to as a "monomer (o)") other than the monomer (m), and for example, is preferably 30% by mass or more, more preferably 40% by mass or more, further preferably 50% by mass or more, still further preferably 55% by mass or more, still more further preferably 60% by mass or more, and even further preferably 65% by mass or more, and for example, is 100% by mass or less, preferably 98% by mass or less, more preferably 95% by mass or less, further preferably 90% by mass or less, still further preferably 85% by mass or less, and still more further preferably 80% by mass or less, based on 100% by mass of the component (A). As described above, the lower limit values and the upper limit values described in a stepwise manner can be independently combined. For example, in one embodiment of the present invention, the content of the monomer (m) in the component (A) is preferably 30 to 100% by mass, more preferably 40 to 98% by mass, further preferably 50 to 95% by mass, still further preferably 55 to 90% by mass, still more further preferably 60 to 85% by mass, and even further preferably 65 to 80% by mass, based on 100% by mass of the component (A).
As described above, the component (A) is a component that is constituted only by the monomer (m) alone, or by the monomer (m) and the monomer (o). The component (A) may contain multiple kinds of the monomer (o). Therefore, the total content of the monomer (m) and the monomer (o) in the component (A) is 100% by mass.

((Meth)acrylic based Polymerizable Monomer)

Examples of the (meth)acrylic based polymerizable monomer constituting the component (A) include a (meth)acrylate ester based polymerizable monomer and a (meth)acrylamide based polymerizable monomer.
Examples of the (meth)acrylic based polymerizable monomer include a polyfunctional (meth)acrylic based polymerizable monomer (a-1) having multiple polymerizable groups (which may be hereinafter referred simply to as a "polyfunctional monomer (a-1)") and a monofunctional (meth)acrylic based polymerizable monomer (a-2) having one polymerizable group (which may be hereinafter referred simply to as a "monofunctional monomer (a-2)".
From the standpoint of achieving more excellent strength and toughness of the denture, it is preferred that the component (A) contains the polyfunctional monomer (a-1), and it is more preferred that the (meth)acrylic based polymerizable monomer (m) contained as a main monomer is the polyfunctional monomer (a-1).

It is preferred that the component (A) contains the monofunctional monomer (a-2), and from the standpoint of achieving more excellent strength and toughness of the denture, it is more preferred that the component (A) contains both the polyfunctional monomer (a-1) and the monofunctional monomer (a-2), and it is further preferred that the component (A) contains the polyfunctional monomer (a-1) as a major monomer and further contains the monofunctional monomer (a-2).

The component (A) may contain one kind of the polyfunctional monomer (a-1) alone, or may contain two or more kinds thereof, and similarly may contain one kind of the monofunctional monomer (a-2) alone, or may contain two or more kinds thereof.

[Polyfunctional (Meth)acrylic based Polymerizable Monomer (a-1)]

The polyfunctional monomer (a-1) is preferably at least one kind selected from the group consisting of a urethanated (meth)acrylate ester compound, an aromatic compound based (meth)acrylate ester compound having no urethane bond, and an alicyclic (meth)acrylate ester compound, more preferably at least one kind selected from a urethanated (meth)acrylate ester compound and an aromatic compound based (meth)acrylate ester compound having no urethane bond, and further preferably a urethanated (meth)acrylate ester compound, from the standpoint of enhancing the strength of the denture. In other words, the component (A) preferably contains at least one kind selected from the group consisting of a urethanated (meth)acrylate ester compound, an aromatic compound based (meth)acrylate ester compound having no urethane bond, and an alicyclic (meth)acrylate ester compound, more preferably contains at least one kind selected from a urethanated (meth)acrylate ester compound and an aromatic compound based (meth)acrylate ester compound having no urethane bond, and further preferably contains a urethanated (meth)acrylate ester compound, as the polyfunctional monomer (a-1).

As described above, the component (A) preferably contains at least one kind selected from the group consisting of a urethanated (meth)acrylate ester compound, an aromatic compound based (meth)acrylate ester compound having no urethane bond, and an alicyclic (meth)acrylate ester compound, which each are the polyfunctional monomer (a-1), more preferably contains at least one kind selected from a urethanated (meth)acrylate ester compound and an aromatic compound based (meth)acrylate ester compound having no urethane bond, and further preferably contains a urethanated (meth)acrylate ester compound, as the (meth)acrylic based polymerizable monomer (m) contained as the major monomer.

The aromatic compound based (meth)acrylate ester compound having no urethane bond more preferably contains at least one kind selected from the group consisting of a hydroxy group, a primary amino group, and a secondary amino group.

It is estimated that these compounds not only accelerate the curing through the dielectric effect of the urethane bond, the aromatic skeleton, the hydroxy group, the primary amino group, and the secondary amino group, but also form a chemical bond, such as a hydrogen bond and a $\pi$-electron effect. Among these, the urethanated (meth)acrylate ester compound having a urethane bond is preferred since the compound has a large effect of imparting toughness to the denture, and thereby breakage and cracks are difficult to occur at the adhesion interface and the coated film, and the resulting denture can exhibit a further excellent flexural strength.

{Urethanated (Meth)acrylate Ester Compound}

The urethanated (meth)acrylate ester compound can be easily synthesized, for example, by subjecting a compound containing one or more kind selected from an alkylene skeleton and a phenylene skeleton and having an isocyanate group, and a (meth)acrylate compound having a hydroxy group (-OH), to addition reaction.

The weight average molecular weight of the urethanated (meth)acrylate ester compound is preferably 1,000 or less from the standpoint of facilitating forming of the composition (I) due to low viscosity thereof and the standpoint of preventing the strength of the denture from being decreased. The weight average molecular weight is more preferably 750 or less, and further preferably 500 or less, from the standpoint of the viscosity of the resulting composition and the strength of the denture. These are also applied to the case where the component (C) described later contains the urethanated (meth)acrylate ester compound from the standpoint that the adhesive composition can be easily coated due to low viscosity, and the penetration of the adhesive composition to the denture base and the prosthetic tooth can be easily achieved, thus the enhancement of the strength of the denture can be facilitated. The weight average molecular weight referred herein means a polystyrene conversion weight average molecular weight obtained by gel permeation chromatography (GPC). In the case where the urethanated (meth)acrylate ester compound does not contain a polymer structure, there is no concept of weight average molecular weight, and therefore the molecular weight thereof is applied.

The urethanated (meth)acrylate ester compound is preferably a compound that does not contain a polymer structure, such as polyester, polycarbonate, polyurethane, and polyether. The absence of the structure contained prevents the concentration of the functional group having polarity from being increased, and facilitates the prevention of the decrease of the strength of the denture in the presence of water, for example, in the oral cavity. In particular, the number of the urethane bond is preferably 3 or less, and more preferably 2 or less, per one molecule, from the standpoint of the strength of the denture in the presence of water. In other words, the number of the urethane bond in the urethanated (meth)acrylate ester compound is preferably 1 to 3 per one molecule, and more preferably 1 or 2 per one molecule.

Examples of the compound containing one or more kind selected from an alkylene skeleton and a phenylene skeleton and having an isocyanate group include methylene diisocyanate (abbr.: MDI), hexamethylene diisocyanate (abbr.: HDI), isophorone diisocyanate (abbr.: IPDI), trimethylhexamethylene diisocyanate (abbr.: TMHMDI), tricyclodecane diisocyanate (abbr.: TCDDI), adamantane diisocyanate (abbr.: ADI), tolylene diisocyanate (abbr.: TDI), xylylene diisocyanate (abbr.: XDI), and diphenylmethane diisocyanate (abbr.: DPMDI). One kind of these compounds may be used alone, or two or more kinds thereof may be used in combination. Among these, HDI, IPDI, TMHMDI, and TCDDI are preferred, and IPDI and TMHMDI are more preferred, from the standpoint of the availability and the prevention of yellowing.

Examples of the (meth)acrylate compound having a hydroxy group include a hydroxy (meth)acrylate compound, such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis(4-(3-(meth)acryloyloxy-2-hydroxypropoxy)phenyl)propane, 1,2-bis(3-(meth)acryloyloxy-2-hydroxypropoxy)ethane, pentaerythritol tri(meth)acrylate, and dipentaerythritol tri- or tetra(meth)acrylate. One kind of these compounds may be used alone, or two or more kinds thereof may be used in combination. Among these, 2-hydroxyethyl (meth)acrylate, 2-hydroxypropyl (meth)acrylate, and 2-hydroxy-3-acryloyloxypropyl (meth)acrylate are preferred, and 2-hydroxyethyl (meth)acrylate is more preferred, from the standpoint of facilitating the exhibition of the excellent strength of the denture.

The addition reaction of the compound containing one or more kind selected from an alkylene skeleton and a phenylene skeleton and containing an isocyanate group and the (meth)acrylate compound having a hydroxy group can be performed according to the ordinary method with no particular limitation.

Examples of the urethanated (meth)acrylate ester compound that does not contain a polymer structure include 2,2,4-trimethylhexamethylenebis(2-carbamoyloxyethyl) dimethacrylate (abbr.: UDMA), 2,4-tolylenebis(2-carbamoyloxyethyl) dimethacrylate, bishydroxyethyl methacrylate-isophorone diurethane, 2,4-tolylenebis(2-carbamoyloxyethyl) dimethacrylate, N,N'-(2,2,4-trimethylhexamethylene)bis[2-(aminocarboxy)propan-1,3-diol] tetramethacrylate, hexamethylenebis(2-carbamoyloxy-3-phenoxypropyl) diacrylate, and 2,4-tolylenebis(2-carbamoyloxyethyl) hexaacrylate. One kind of these compounds may be used alone, or two or more kinds thereof may be used in combination. Among these, 2,2,4-trimethylhexamethylenebis(2-carbamoyloxyethyl) dimethacrylate and N,N'-(2,2,4-trimethylhexamethylene)bis[2-(aminocarboxy)propan-1,3-diol] tetramethacrylate are preferred, and 2,2,4-trimethylhexamethylenebis(2-carbamoyloxyethyl) dimethacrylate is more preferred, from the standpoint of enhancing the strength of the denture.

In the case where the component (A) contains the urethanated (meth)acrylate ester compound, which is the polyfunctional monomer (a-1), the content of the urethanated (meth)acrylate ester compound in the component (A) is preferably 10 to 100% by mass based on 100% by mass of the component (A), and is more preferably 30 to 95% by mass, further preferably 50 to 90% by mass, and still further preferably 60 to 80% by mass, from the standpoint of achieving the further excellent strength of the denture.

In one embodiment of the present invention, in the case where the component (A) contains multiple polyfunctional monomers (a-1), the content of the urethanated (meth)acrylate ester compound is preferably 50 to 100% by mass, more preferably 60 to 100% by mass, further preferably 70 to 100% by mass, still further preferably 80 to 100% by mass, still more further preferably 90 to 100% by mass, and even further preferably 95 to 100% by mass, based on 100% by mass in total of the polyfunctional monomers (a-1).

{Aromatic Compound based (Meth)acrylate Ester Compound containing No Urethane Bond}

Examples of the aromatic compound based (meth)acrylate ester compound containing no urethane bond include 2,2-bis((meth)acryloyloxyphenyl)propane, 2,2-bis(4-(3-acryloyloxy)-2-hydroxypropoxyphenyl)propane, 2,2-bis(4-(3-methacryloyloxy-2-hydroxypropoxy)phenyl)propane (abbr.: Bis-GMA), 2,2-bis(4-(2-(3-acryloyloxy-2-hydroxypropoxy)propyl)phenyl)propane, 2,2-bis(4-(2-(3-methacryloyloxy-2-hydroxypropoxy)propyl)phenyl)propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl) pyromellitate. One kind of these compounds may be used alone, or two or more kinds thereof may be used in combination. Among these, a compound containing a hydroxy group is preferred, and specifically 2,2-bis(4-(3-acryloyloxy)-2-hydroxypropoxyphenyl)propane, 2,2-bis(4-(3-methacryloyloxy-2-hydroxypropoxy)phenyl)propane, 2,2-bis(4-(2-(3-acryloyloxy-2-hydroxypropoxy)propyl)phenyl)propane, 2,2-bis(4-(2-(3-methacryloyloxy-2-hydroxypropoxy)propyl)phenyl)propane are preferred, from the standpoint of enhancing the strength of the denture. Among these, 2,2-bis(4-(3-methacryloyloxy-2-hydroxypropoxy)phenyl)propane is more preferred.

In the case where the component (A) contains the aromatic compound based (meth)acrylate ester compound containing no urethane bond, which is the polyfunctional monomer (a-1), the content of the aromatic compound based (meth)acrylate ester compound containing no urethane bond in the component (A) is preferably 5 to 95% by mass based on 100% by mass of the component (A), and is more preferably 10 to 90% by mass, further preferably 20 to 85% by mass, still further preferably 30 to 80% by mass, and still more further preferably 50 to 70% by mass, from the standpoint of achieving the further excellent strength of the denture.

In one embodiment of the present invention, the content of the aromatic compound based (meth)acrylate ester compound containing no urethane bond is preferably 50 to 100% by mass, more preferably 60 to 100% by mass, further preferably 70 to 100% by mass, still further preferably 80 to 100% by mass, still more further preferably 90 to 100% by mass, and even further preferably 95 to 100% by mass, based on 100% by mass in total of the polyfunctional monomer (a-1).

{Alicyclic (Meth)acrylate Ester Compound)

Examples of the alicyclic (meth)acrylate ester compound include tricyclodecanedimethanol di(meth)acrylate, cyclohexanediol di(meth)acrylate, and adamantanediol di(meth)acrylate. Among these, tricyclodecanedimethanol di(meth)acrylate is preferred.

In the case where the component (A) contains the alicyclic (meth)acrylate ester compound, which is the polyfunctional monomer (a-1), the content of the alicyclic (meth)acrylate ester compound in the component (A) is preferably 30 to 100% by mass based on 100% by mass of the component (A), and is more preferably 50 to 98% by mass, and further preferably 70 to 95% by mass, from the standpoint of achieving the further excellent strength of the denture.

In one embodiment of the present invention, the content of the alicyclic (meth)acrylate ester compound is preferably 50 to 100% by mass, more preferably 60 to 100% by mass, further preferably 70 to 100% by mass, still further preferably 80 to 100% by mass, still more further preferably 90 to 100% by mass, and even further preferably 95 to 100% by mass, based on 100% by mass in total of the polyfunctional monomer (a-1).

{Additional Polyfunctional (Meth)acrylate Ester based Polymerizable Monomer}

Examples of a polyfunctional (meth)acrylate ester based polymerizable monomer other than the urethanated (meth)acrylate ester compound, the aromatic compound based (meth)acrylate ester compound containing no urethane bond, and the alicyclic (meth)acrylate ester compound, i.e., an additional polyfunctional (meth)acrylate ester based polymerizable monomer (which may be hereinafter referred to as an "additional polyfunctional monomer"), include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, and dipentaerythritol penta(meth)acrylate. Among these, glycerol di(meth)acrylate, 1,2-bis(3-methacryloyloxy-2-hydroxypropoxy)ethane, and triethylene glycol di(meth)acrylate are preferred, and triethylene glycol di(meth)acrylate is more preferred, from the standpoint of achieving the further excellent strength of the denture. One kind of these compounds may be used alone, or two or more kinds thereof may be used in combination.

In the case where the component (A) contains the additional polyfunctional (meth)acrylate ester based polymerizable monomer, the content of the additional polyfunctional (meth)acrylate ester based polymerizable monomer in the component (A) is preferably 1 to 50% by mass, more preferably 5 to 40% by mass, and further preferably 8 to 35% by mass, based on 100% by mass of the component (A).

In one embodiment of the present invention, in the case where the component (A) contains the additional polyfunctional (meth)acrylate ester based polymerizable monomer, the content of the additional polyfunctional (meth)acrylate ester based polymerizable monomer is preferably 1 to 50% by mass, more preferably 1 to 40% by mass, further preferably 1 to 35% by mass, and still further preferably 1 to 30% by mass, based on 100% by mass in total of the polyfunctional monomer (a-1).

In the case where the component (A) contains the polyfunctional monomer (a-1), the total content of the polyfunctional monomer (a-1) in the component (A) is preferably 10 to 100% by mass based on 100% by mass of the component (A), and is more preferably 50 to 100% by mass, further preferably 50 to 95% by mass, still further preferably 55 to 85% by mass, and still more further preferably 60 to 80% by mass, from the standpoint of achieving the further excellent strength and toughness of the denture.

{Monofunctional (Meth)acrylic based Polymerizable Monomer (a-2)}

As described above, the component (A) preferably contains the monofunctional monomer (a-2). The monofunctional monomer (a-2) lowers the viscosity of the composition (I), and also decreases the crosslinking density of the resulting stereolithography article, thereby enhancing the toughness of the denture.

The one polymerizable group of the monofunctional monomer (a-2) means a (meth)acryloyl group.

Examples of the monofunctional monomer (a-2) include a (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, a linear (meth)acrylate ester based polymerizable monomer, a cyclic (meth)acrylamide compound, and a linear (meth)acrylamide compound. From the standpoint of preventing an offensive odor in handling, a (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, and a cyclic (meth)acrylamide compound, which tend to have a higher boiling point, preventing an odor, are preferred, and among these, a (meth)acrylate ester based polymerizable monomer containing an aromatic ring is preferred from the standpoint of enhancing the strength of the denture. In other words, the component (A) preferably contains at least one kind selected from the group consisting of a (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, and a cyclic (meth)acrylamide compound, and more preferably contains a (meth)acrylate ester based polymerizable monomer containing an aromatic ring, as the monofunctional monomer (a-2).

A (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, and a cyclic (meth)acrylamide compound tend to have a higher boiling point and to prevent an odor as compared to a linear (meth)acrylate ester based polymerizable monomer, assuming the equivalent molecular weights therefor, and the tendency is estimated to derive from the $\pi$-electron interaction and the effect of imparting minute polarity through the fixed steric conformation.

Examples of the (meth)acrylate ester based polymerizable monomer containing an aromatic ring include o-phenylphenol (meth)acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth)acrylate, butoxylated o-phenylphenol (meth)acrylate, butoxylated m-phenylphenol (meth)acrylate, butoxylated p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, phenyl (meth)acrylate, 4-biphenylyl (meth)acrylate, 1-naphthyl (meth)acrylate, 2-naphthyl (meth)acrylate, anthryl (meth)acrylate, o-2-propenylphenyl (meth)acrylate, benzhydrol (meth)acrylate, cumylphenol (meth)acrylate, fluorenyl (meth)acrylate, and fluorenylmethyl (meth)acrylate.

Examples of the alicyclic (meth)acrylate ester based polymerizable monomer include 2-(1-adamantyl)propyl (meth)acrylate, 2-methyladamantyl-2-yl (meth)acrylate, 2-ethyladamantyl-2-yl (meth)acrylate, 2-n-propyladamantyl-2-yl (meth)acrylate, 2-isopropyladamantyl-2-yl (meth)acrylate, 1-(adamantan-1-yl)-1-methylethyl (meth)acrylate, 1-(adamantan-1-yl)-1-ethylethyl (meth)acrylate, 1-(adamantan-1-yl)-1-methylpropyl (meth)acrylate, and 1-(adamantan-1-yl)-1-ethylpropyl (meth)acrylate.

Examples of the cyclic (meth)acrylate ester based polymerizable monomer containing a nitrogen atom include pentamethylpiperidinyl (meth)acrylate, tetramethylpiperidinyl (meth)acrylate, and 4-(pyrimidin-2-yl)piperazin-1-yl (meth)acrylate.

Examples of the linear (meth)acrylate ester based polymerizable monomer include 2-ethylhexyl (meth)acrylate, decyl (meth)acrylate, undecyl (meth)acrylate, lauryl (meth)acrylate, tridecyl (meth)acrylate, tetradecyl (meth)acrylate, pentadecyl (meth)acrylate, cetyl (meth)acrylate, palmitoleyl (meth)acrylate, heptadecyl (meth)acrylate, oleyl (meth)acrylate, stearyl (meth)acrylate, isostearyl (meth)acrylate, 4-hydroxybutyl (meth)acrylate, 6-hydroxyhexyl (meth)acrylate, 10-hydroxydecyl (meth)acrylate, glycerol mono(meth)acrylate, erythritol mono(meth)acrylate, and isobornylcyclohexyl (meth)acrylate.

Examples of the cyclic (meth)acrylamide compound include N-(meth)acryloylmorpholine, N-(meth)acryloylpyrrolidine, N-(meth)acryloylpiperidine, N-(meth)acryloyl-2-methylpiperidine, and N-(meth)acryloyl-2,2,6,6-tetramethylpiperidine.

Examples of the linear (meth)acrylamide compound include N,N-dimethyl(meth)acrylamide, N,N-diethyl(meth)acrylamide, N,N-di-n-propyl(meth)acrylamide, N-isopropyl(meth)acrylamide, N,N-di-n-butyl(meth)acrylamide, N,N-di-n-hexyl(meth)acrylamide, N,N-di-n-octyl(meth)acrylamide, N,N-di-2-ethylhexyl(meth)acrylamide, N-(2-hydroxyethyl)(meth)acrylamide, and N,N-bis(2-hydroxyethyl)(meth)acrylamide.

Among these, the monofunctional monomer (a-2) is preferably o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, o-phenylphenol (meth)acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, fluorenyl (meth)acrylate, fluorenylmethyl (meth)acrylate, and 4-biphenylyl (meth)acrylate, more preferably m-phenoxybenzyl (meth)acrylate, o-phenoxybenzyl (meth)acrylate, p-phenoxybenzyl (meth)acrylate, o-phenylphenol (meth)acrylate, m-phenylphenol (meth)acrylate, p-phenylphenol (meth)acrylate, ethoxylated o-phenylphenol (meth)acrylate, ethoxylated m-phenylphenol (meth)acrylate, and ethoxylated p-phenylphenol (meth)acrylate, further preferably m-phenoxybenzyl (meth)acrylate and ethoxylated o-phenylphenol (meth)acrylate, and still further preferably m-phenoxybenzyl acrylate and ethoxylated o-phenylphenol acrylate, from the standpoint of enhancing the curability of the resulting composition and enhancing the strength of the denture.

In the case where the component (A) contains the monofunctional monomer (a-2), the total content of the monofunctional monomer (a-2) in the component (A) is preferably 1 to 60% by mass based on 100% by mass of the component (A), and is preferably 5 to 50% by mass, more preferably 10 to 50% by mass, further preferably 15 to 45% by mass, and still further preferably 20 to 40% by mass from the standpoint of achieving the further excellent strength and toughness of the denture.

In one embodiment of the present invention, the content of the (meth)acrylate ester based monomer containing an aromatic ring is preferably 50 to 100% by mass, more preferably 60 to 100% by mass, further preferably 70 to 100% by mass, still further preferably 80 to 100% by mass, still more further preferably 90 to 100% by mass, and even further preferably 95 to 100% by mass, based on 100% by mass in total of the monofunctional monomer (a-2).

In one embodiment of the present invention, the (meth)acrylic based polymerizable monomer contained in the component (A) may be constituted substantially only by the polyfunctional monomer (a-1), or the polyfunctional monomer (a-1) and the monofunctional monomer (a-2). The fact that the (meth)acrylic based polymerizable monomer is constituted substantially only by the polyfunctional monomer (a-1), or the polyfunctional monomer (a-1) and the monofunctional monomer (a-2) means the total content of the additional (meth)acrylic based polymerizable monomer other than the polyfunctional monomer (a-1), or the polyfunctional monomer (a-1) and the monofunctional monomer (a-2) is less than 10.0% by mass, preferably less than 5.0% by mass, more preferably less than 1.0% by mass, further preferably less than 0.1% by mass, and particularly preferably less than 0.01% by mass, based on 100% by mass as the total mass of the component (A).

In one embodiment of the present invention, the total content of the polyfunctional monomer (a-1) and the monofunctional monomer (a-2) in the component (A) is preferably 80 to 100% by mass, more preferably 90 to 100% by mass, further preferably 95 to 100% by mass, still further preferably 99 to 100% by mass, still more further preferably 99.9 to 100% by mass, even further preferably 99.99 to 100% by mass, based on 100% by mass of the component (A).

The content of the component (A) in the curable composition for a denture base (I) is preferably 40% by mass or more, more preferably 45% by mass or more, further preferably 50% by mass or more, still further preferably 60% by mass or more, still more further preferably 80% by mass or more, and even further preferably 90% by mass or more, and is preferably 99.99% by mass or less, more preferably 99.5% by mass or less, further preferably 99% by mass or less, and still further preferably 98% by mass or less, based on 100% by mass of the composition (I), from the standpoint of achieving the further excellent strength and toughness of the denture. As described above, the lower limit values and the upper limit values described in a stepwise manner can be independently combined. For example, in one embodiment of the present invention, the content of the component (A) in the curable composition for a denture base (I) is preferably 40 to 99.99% by mass, more preferably 45 to 99.99% by mass, further preferably 50 to 99.5% by mass, still further preferably 60 to 99.5% by mass, still more further preferably 80 to 99% by mass, and even further preferably 90 to 98% by mass, based on 100% by mass of the composition (I).

<Photopolymerization Initiator>

The photopolymerization initiator may be selected from polymerization initiators that have been used in the ordinary industries, and among these, a photopolymerization initiator used in the dental field is preferred.

Examples of the photopolymerization initiator include a (bis)acylphosphine oxide compound, a thioxanthone compound or a quaternary ammonium salt of a thioxanthone compound, a ketal compound, an $\alpha$-diketone compound, a coumarin compound, an anthraquinone compound, a benzoin alkyl ether compound, and an $\alpha$-aminoketone based compound. One kind of the photopolymerization initiator may be used alone, or two or more kinds thereof may be used in combination.

Among these photopolymerization initiators, at least one kind selected from the group consisting of a (bis)acylphosphine oxide compound and an $\alpha$-diketone compound is preferably used. With the use thereof, the composition that is excellent in photocurability in the ultraviolet region and the visible light region, and shows the sufficient photocurability by using any of light sources including a laser, a halogen lamp, a light emitting diode (LED), and a xenon lamp.

In the (bis)acylphosphine oxide compound, examples of the acylphosphine oxide compound include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyldi-(2,6-dimethylphenyl) phosphonate, 2,4,6-trimethylbenzoylphenylphosphine oxide sodium salt, 2,4,6-trimethylbenzoylphenylphosphine oxide potassium salt, and 2,4,6-trimethylbenzoyldiphenylphosphine oxide ammonium salt.

In the (bis)acylphosphine oxide compound, examples of the bisacylphosphine oxide compound include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide. Examples thereof also include the compounds described in JP2000-159621A.

Among the (bis)acylphosphine oxide compounds, at least one kind selected from the group consisting of 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and 2,4,6-trimethylbenzoylphenylphosphine oxide sodium salt is preferably used as the photopolymerization initiator.

Examples of the $\alpha$-diketone compound include diacetyl, benzyl, camphorquinone, 2,3-pentadione, 2,3-octadione, 9,10-phenanthrenequinone, 4,4'-oxybenzyl, and acenaphthenequinone. Among these $\alpha$-diketone compounds, camphorquinone is preferred in the case where a light source in the visible light region is used.

While the content of the photopolymerization initiator in the composition (I) is not particularly limited, the photopolymerization initiator is preferably 0.01 to 20 parts by mass per 100 parts by mass of the component (A) from the standpoint of the curability of the resulting composition and the like. In the case where the content of the photopolymerization initiator is 0.01 part by mass or more, the polymerization can be allowed to proceed sufficiently. From this standpoint, the content of the photopolymerization initiator in the composition (I) is more preferably 0.05 part by mass or more, further preferably 0.1 part by mass or more, still further preferably 0.5 part by mass or more, and still more further preferably 1.0 part by mass or more, per 100 parts by mass of the component (A). In the case where the content of the photopolymerization initiator is 20 parts by mass or less, the photopolymerization initiator can be prevented from being deposited from the composition even in the case where the solubility thereof is low. From this standpoint, the content of the photopolymerization initiator in the composition (I) is more preferably 15 parts by mass or less, more preferably 10 parts by mass or less, and further preferably 5.0 part by mass or less, per 100 parts by mass of the component (A). As described above, the lower limit values and the upper limit values described in a stepwise manner can be independently combined. For example, in one embodiment of the present invention, the content of the photopolymerization initiator in the composition (I) is preferably 0.01 to 20 parts by mass, more preferably 0.05 to 15 parts by mass, further preferably 0.1 to 10 parts by mass, still further preferably 0.5 to 5.0 parts by mass, and still more further preferably 1.0 to 5.0 parts by mass, per 100 parts by mass of the component (A).

<Additional Components>

The curable composition for a denture base (I) may contain a component other than the component (A) and the photopolymerization initiator depending on necessity in such a range that does not impair the spirit of the present invention. The composition (I) can be produced according to a known method.

(Colorant)

The curable composition for a denture base (I) may contain a colorant, for example, from the standpoint of regulating the color of the denture base to the gingival color or the like. The method of regulating the color of the composition (I) is not particularly limited, and the color thereof can be regulated, for example, by mixing known colorants.

The colorant used may be, for example, a pigment or a dye. The pigment can be roughly classified into an inorganic pigment and an organic pigment, which may be mixed in use.

Examples of the inorganic pigment include an oxide, a hydroxide, a sulfide, a chromate salt, a silicate salt, a sulfate salt, a carbonate salt, a ferrocyanic compound, a phosphate salt, and carbon, and among these, an oxide is preferably used. Specific examples of the oxide include red iron oxide, yellow iron oxide, black iron oxide, titanium oxide ($TiO_2$), alumina, and zirconia. The red iron oxide is colcothar or hematite and is iron oxide shown by the chemical formula $Fe_2O_3$, yellow iron oxide is goethite and is iron oxide shown by the chemical formula $FeOOH$, and black iron oxide is magnetite and is iron oxide shown by the chemical formula $Fe_3O_4$.

Examples of the organic pigment include an azo based pigment, a phthalocyanine pigment, a condensed polycyclic pigment, a nitro based pigment, a nitroso based pigment, and a fluorescent pigment, and among these, an azo based pigment and a phthalocyanine pigment are preferably used. Specific examples of the azo based pigment include Novoperm Yellow 4G, Chromophthal Yellow 3G, Chromophthal Yellow 3RLP, Novoperm Red BN, Chromophthal Scarlet RN, Chromophthal Blue 4GNP, and Blue No.2 aluminum lake. Specific examples of the phthalocyanine pigment include Phthalocyanine Blue and Phthalocyanine Green.

In one embodiment of the present invention, the composition (I) preferably satisfies the following condition (1).

Condition (1): The chromaticity on white background according to the L*a*b* color coordinate system of a test piece having a diameter of 15.0 ± 0.1 mm and a thickness of 1.00 ± 0.01 mm formed of a cured product of the curable composition for a denture base (I) is 70.0 ≤ L*white ≤ 90.0, 10.0 ≤ a*white ≤ 25.0 (preferably 10.0 < a*white ≤ 25.0), 10.0 ≤ b*white ≤ 35.0, and 10.0 ≤ ΔL ≤ 40.0.

For example, in the case where the color of the composition (I) is to be regulated to satisfy the range of the condition (1), preferred examples of the pigment that can be used mainly therefor include red iron oxide.

In one embodiment of the present invention, the brightness difference ΔL between the brightness L*black obtained by measuring on black background and the brightness L*white obtained by measuring on white background according to the L*a*b* color coordinate system of a test piece having a diameter of 15.0 ± 0.1 mm and a thickness of 1.00 ± 0.01 mm formed of a cured product of the curable composition for a denture base (I) is preferably 10.0 or more and 40.0 or less, more preferably more than 20.0 and 40.0 or less, and further preferably 24.0 or more and 40.0 or less.

In one embodiment of the present invention, the value of a*white obtained by measuring on white background according to the L*a*b* color coordinate system of a test piece having a diameter of 15.0 ± 0.1 mm and a thickness of 1.00 ± 0.01 mm formed of a cured product of the curable composition for a denture base (I) is preferably larger than the value of a*white obtained by measuring on white background according to the L*a*b* color coordinate system of a test piece having a diameter of 15.0 ± 0.1 mm and a thickness of 1.00 ± 0.01 mm formed of a cured product of the composition for a prosthetic tooth (II).

In the case where the composition (I) contains the colorant, the total content of the colorant in the composition (I) is not particularly limited, and for example, is preferably 0.0005 to 5.0 parts by mass per 100 parts by mass of the component (A) from the standpoint of the color of the denture base and the toughness of the denture. The total content of the colorant in the composition (I) is preferably 0.0005 part by mass or more since a desired color can be easily imparted thereby. The total content of the colorant in the composition (I) is preferably 5.0 parts by mass or less since the influence thereof on the curability of the composition (I) can be suppressed, and the toughness of the denture can be prevented from being decreased thereby. From this standpoint, the total content of the colorant in the composition (I) is more preferably 0.0008 part by mass or more, and further preferably 0.001 part by mass or more, and is more preferably 1.0 part by mass or less, and further preferably 0.5 part by mass or less, per 100 parts by mass of the component (A). As described above, the lower limit values and the upper limit values described in a stepwise manner can be independently combined. For example, in one embodiment of the present invention, in the case where the composition (I) contains the colorant, the total content of the colorant in the composition (I) is preferably 0.0005 to 5.0 parts by mass, more preferably 0.0008 to 1.0 part by mass, and further preferably 0.001 to 0.5 part by mass, per 100 parts by mass of the component (A).

In the case where the colorant is a pigment, the preferred ranges of the total content thereof are the same as the total content of the colorant.

(Polymerization Accelerator)

The composition (I) may contain a polymerization accelerator in a range that does not impair the spirit of the present invention, for the purpose of enhancing the photocuring capability. Examples of the polymerization accelerator include ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. Among these, at least one selected from the group consisting of ethyl 4-(N,N-dimethylamino)benzoate, n-butoxyethyl 4-(N,N-dimethylamino)benzoate, and 4-(N,N-dimethylamino)benzophenone is preferably used from the standpoint of imparting the excellent curability to the composition (I). One kind of the polymerization initiator may be used alone, or two or more kinds thereof may be used in combination.

(Filler)

The composition (I) may contain a filler from the standpoint of regulating the viscosity of the composition, imparting the strength and the antibacterial activity to the denture base, and the like. Examples of the filler include an organic filler, an inorganic filler, and an organic-inorganic composite filler. One kind of the filler may be used alone, or two or more kinds thereof may be used in combination.

Examples of the material of the organic filler include polymethyl methacrylate, polyethyl methacrylate, a methyl methacrylate-ethyl methacrylate copolymer, crosslinked polymethyl methacrylate, crosslinked polyethyl methacrylate, polyester, polyamide, polycarbonate, polyphenylene ether, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. One kind of these materials may be used alone, or two or more kinds thereof may be used in combination. The shape of the organic filler is not particularly limited, and the particle diameter of the filler can be appropriately selected in use.

Examples of the material of the inorganic filler include quartz, silica, alumina, silica-titania, silica-titania-barium oxide, silica-zirconia, silica-alumina, lanthanum glass, borosilicate glass, soda glass, barium glass, strontium glass, glass ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. One kind of these materials may be used alone, or two or more kinds thereof may be used in combination. The shape of the inorganic filler is not particularly limited, and an irregular shape filler or a spherical filler can be appropriately selected in use.

Examples of the organic-inorganic composite filler include an organic-inorganic composite filler obtained by dispersing an inorganic based filler in an organic based filler, and an organic-inorganic composite filler obtained by coating an inorganic based filler with various kinds of a polymerizable monomer.

In the case where the composition (I) contains the filler, for example, the content of the filler in the composition (I) is preferably 0.1 part by mass or more, more preferably 0.3 part by mass or more, and further preferably 0.5 part by mass or more, per 100 parts by mass of the component (A), from the standpoint of regulating the viscosity of the composition, imparting the strength to the denture base, and the like. The content of the filler in the composition (I) is preferably 20 parts by mass or less, more preferably 10 parts by mass or less, and further preferably 5 parts by mass or less, per 100 parts by mass of the component (A), from the standpoint of suppressing the influence thereof on formability due to the viscosity of the composition (I) increase, and the standpoint of achieving the excellent strength and toughness of the denture. As described above, the lower limit values and the upper limit values described in a stepwise manner can be independently combined. For example, in one embodiment of the present invention, in the case where the composition (I) contains the filler, the content of the filler in the composition (I) is preferably 0.1 to 20 parts by mass, more preferably 0.3 to 10 parts by mass, and further preferably 0.5 to 5 parts by mass, per 100 parts by mass of the component (A).

(Polymer)

The composition (I) may contain a polymer for the purpose of modifying the flexibility, the flowability, and the like, in such a range that does not impair the spirit of the present invention. For example, natural rubber, synthetic polyisoprene rubber, liquid polyisoprene rubber or a hydrogenated product thereof, polybutadiene rubber, liquid polybutadiene rubber or a hydrogenated product thereof, styrene-butadiene rubber, chloroprene rubber, ethylenepropylene rubber, acrylic rubber, isoprene-isobutylene rubber, acrylonitrile-butadiene rubber, or a styrene based elastomer may be added. Specific examples of other polymers that can be added include a polystyrene-polyisoprene-polystyrene block copolymer, a polystyrene-polybutadiene-polystyrene block copolymer, a poly($\alpha$-methylstyrene)-polybutadiene-poly($\alpha$-methylstyrene) block copolymer, a poly(p-methylstyrene)-polybutadiene-poly(p-methylstyrene) block copolymer, and hydrogenated products thereof. One kind of the polymer may be used alone, or two or more kinds thereof may be used in combination.

(Softener)

The composition (I) may contain a softener depending on necessity. Examples of the softener include a petroleum based softener, such as paraffin based, naphthene based, and aromatic based process oils, and a vegetable oil based softener, such as paraffin, peanut oil, and rosin. One kind of the softener may be used alone, or two or more kinds thereof may be used in combination. In the case where the softener is used, the content of the softener in the composition (I) is not particularly limited unless the spirit of the present invention is impaired, and is generally preferably 1 to 30 parts by mass, and more preferably 5 to 20 parts by mass, per 100 parts by mass of the component (A).

(Stabilizer)

The composition (I) may contain a known stabilizer for the purpose of preventing the deterioration or regulating the photocurability. Examples of the stabilizer include a polymerization inhibitor, an ultraviolet ray absorbent, and an antioxidant.

Examples of the polymerization inhibitor include hydroquinone, hydroquinone monomethyl ether, dibutylhydroquinone, dibutylhydroquinone monomethyl ether, 4-t-butylcatechol, 2-t-butyl-4,6-dimethylphenol, 2,6-di-t-butylphenol, and 3,5-di-t-butyl-4-hydroxytoluene. The content of the polymerization inhibitor is preferably 0.001 to 5.0 parts by mass, more preferably 0.01 to 3.0 parts by mass, and further preferably 0.1 to 2.0 parts by mass, per 100 parts by mass of the component (A).

(Additional Additive)

The composition (I) may contain a known additional additive depending on necessity, for example, for regulating the paste properties, in addition to the components described above in such a range that does not impair the spirit of the present invention. Examples of the additional additive include a thickener, a prepolymer (oligomer), and an aryl compound (such as an aryl iodonium salt). One kind of the additional additive may be used alone, or two or more kinds thereof may be used in combination.

[Composition for Prosthetic Tooth (II)]

The composition for a prosthetic tooth (II) (which may be hereinafter referred simply to as a "composition (II)") contains a (meth)acrylic based polymerizable monomer component (B) (which may be hereinafter referred simply to as a "component (B)") containing the same (meth)acrylic based polymerizable monomer (m) as contained in the curable composition for a denture base (I).

The composition (II) preferably has curability, and more preferably has photocurability.

<(Meth)acrylic based Polymerizable Monomer Component (B)>

The (meth)acrylic based polymerizable monomer component (B) is a component that is constituted by one kind of a (meth)acrylic based polymerizable monomer alone or two or more kinds thereof, and contains the same monomer (m) as contained in the composition (I) in an amount of 20% by mass or more based on 100% by mass of the component (B).

Accordingly, the component (B) necessarily contains the same (meth)acrylic based polymerizable monomer as the monomer (m), which is the (meth)acrylic based polymerizable monomer contained as the major monomer in the (meth)acrylic based polymerizable monomers constituting the component (A), in an amount of 20% by mass or more based on 100% by mass of the component (B).

The "same (meth)acrylic based polymerizable monomer as the monomer (m)" herein means the sameness as the monomer (m) in terms of specific compound. For example, in the case where the monomer (m) in the component (A) is UDMA, the expression in the component (B) means that the monomer (m) that is necessarily contained in an amount of 20% by mass or more based on 100% by mass of the component (B) is UDMA in question.

Therefore, for example, in the case where the monomer (m) in the component (A) is UDMA, which is the polyfunctional monomer (a-1), the fact that the component (B) contains Bis-GMA, which is in the same category as the polyfunctional monomer (a-1), in an amount of 20% by mass or more based on 100% by mass of the component (B) does not satisfy the requirement that the "same (meth)acrylic based polymerizable monomer as the monomer (m)" is contained.

In the case where multiple kinds of the monomers (m) as the major monomers exist in the component (A), for example, in the case where two kinds of the (meth)acrylic based polymerizable monomers are contained each in an amount of 50% by mass in the component (A), it suffices that the component (B) contains any one of the monomers (m) in the component (A) in an amount of 20% by mass or more, and it is not necessary that all the monomers (m) in the component (A) are contained in an amount of 20% by mass or more.

The content of the monomer (m) in the component (B) is preferably 30% by mass or more, more preferably 40% by mass or more, further preferably 50% by mass or more, still further preferably 55% by mass or more, still more further preferably 60% by mass or more, and even further preferably 65% by mass or more, and is for example, 100% by mass or less, preferably 98% by mass or less, more preferably 95% by mass or less, further preferably 90% by mass or less, still further preferably 85% by mass or less, and still more further preferably 80% by mass or less, based on 100% by mass of the component (B), from the standpoint of achieving more excellent strength and toughness of the denture. As described above, the lower limit values and the upper limit values described in a stepwise manner can be independently combined. For example, in one embodiment of the present invention, the content of the monomer (m) in the component (B) is preferably 20 to 100% by mass, more preferably 30 to 100% by mass, further preferably 40 to 98% by mass, still further preferably 50 to 95% by mass, still more further preferably 55 to 90% by mass, even further preferably 60 to 85% by mass, and even still further preferably 65 to 80% by mass, based on 100% by mass of the component (B).

The (meth)acrylic based polymerizable monomer used for constituting the component (B) may be the similar (meth)acrylic based polymerizable monomer as described above for the component (A), and the preferred kinds of the (meth)acrylic based polymerizable monomer therefor are also the same as described for the component (A). The content of the (meth)acrylic based polymerizable monomers that the component (B) may contain and the preferred ranges thereof are also the same as described for the component (A) except that the description "based on 100% by mass of the component (A)" is changed to "based on 100% by mass of the component (B)", as far as satisfying the requirement to contain the monomer (m) in an amount of 20% by mass or more based on 100% by mass of the component (B). The reasons for the preferred embodiments and the preferred ranges thereof are also the same as described above unless otherwise indicated.

As described above, the component (B) is a component that is constituted only by the monomer (m) alone, or by the monomer (m) and the monomer (o). The component (B) may contain multiple kinds of the monomer (o). Therefore, the total content of the monomer (m) and the monomer (o) in the component (B) is 100% by mass.

In one embodiment of the present invention, it is preferred that not only the component (B) contains the same monomer (m) as the component (A), but also the monomer (o) contained therein is the same (meth)acrylic based polymerizable monomer as the monomer (o) contained in the component (A). The "same (meth)acrylic based polymerizable monomer as the monomer (o)" herein is the same as the description for the "same (meth)acrylic based polymerizable monomer as the monomer (m)" above. In the case where the component (A) contains multiple compounds as the monomer (o), the component (B) preferably contains all the multiple compounds as the monomer (o).

In one embodiment of the present invention, furthermore, the absolute value of the difference between the content of the monomer (m) in the component (B) and the content of the monomer (m) in the component (A) is preferably 10% by mass or less, more preferably 5% by mass or less, further preferably 3% by mass or less, and still further preferably 1% by mass or less. In other words, in one embodiment of the present invention, the absolute value of the difference between the content of the monomer (m) in the component (B) and the content of the monomer (m) in the component (A) is preferably 0 to 10% by mass, more preferably 0 to 5% by mass, further preferably 0 to 3% by mass, and still further preferably 0 to 1% by mass.

Similarly, the absolute value of the difference between the content of the monomer (o) in the component (B) and the content of the monomer (o) in the component (A) is preferably 10% by mass or less, more preferably 5% by mass or less, further preferably 3% by mass or less, and still further preferably 1% by mass or less. In other words, in one embodiment of the present invention, the absolute value of the difference between the content of the monomer (o) in the component (B) and the content of the monomer (o) in the component (A) is preferably 0 to 10% by mass, more preferably 0 to 5% by mass, further preferably 0 to 3% by mass, and still further preferably 0 to 1% by mass. In the case where the component (A) contains multiple compounds as the monomer (o), and the component (B) also contains the same multiple compounds as in the component (A) as the monomer (o), the absolute values of the differences between the content in the component (A) and the content in the component (B) of the corresponding compounds each are independently preferably 10% by mass or less, more preferably 5% by mass or less, further preferably 3% by mass or less, and still further preferably 1% by mass or less. In other words, in one embodiment of the present invention, in the case where the component (A) contains multiple compounds as the monomer (o), and the component (B) also contains the same multiple compounds as in the component (A) as the monomer (o), the absolute values of the differences between the content in the component (A) and the content in the component (B) of the corresponding compounds each are independently preferably 0 to 10% by mass, more preferably 0 to 5% by mass, further preferably 0 to 3% by mass, and still further preferably 0 to 1% by mass.

However, as described above, the total content of the monomer (m) and the monomer (o) in the component (B) is 100% by mass.

In one embodiment of the present invention, the component (B) preferably contains the (meth)acrylic based polymerizable monomer component (A) in an amount of 70% by mass or more, more preferably 80% by mass or more, and further preferably 90% by mass or more, and in an amount of 100% by mass or less, based on 100% by mass of the (meth)acrylic based polymerizable monomer component (B), from the standpoint of achieving more excellent strength and toughness of the denture. In other words, in one embodiment of the present invention, the component (B) preferably contains the (meth)acrylic based polymerizable monomer component (A) in an amount of 70 to 100% by mass, more preferably 80 to 100% by mass, and further preferably 90 to 100% by mass, based on 100% by mass of the (meth)acrylic based polymerizable monomer component (B). In the embodiment of the present invention, the component (B) still further preferably contains the (meth)acrylic based polymerizable monomer component (A) in an amount of 100% by mass based on 100% by mass of the (meth)acrylic based polymerizable monomer component (B). In other words, it is still further preferred to use the component (A) as the component (B).

In one embodiment of the present invention, the composition of the component (B) (the "composition" herein means the "combination of the (meth)acrylic based polymerizable monomers and the content ratio (mass ratio) thereof", which is hereinafter similarly applied to the "composition of the component (A)" and the "composition of the component (C)") is still more further preferably the same as the composition of the component (A).

The content of the component (B) in the composition for a prosthetic tooth (II) is preferably 40% by mass or more, more preferably 45% by mass or more, further preferably 50% by mass or more, still further preferably 60% by mass or more, still more further preferably 80% by mass or more, even further preferably 90% by mass or more, and even still further preferably 95% by mass or more, and is 100% by mass or less, preferably 99.99% by mass or less, more preferably 99.5% by mass or less, further preferably 99% by mass or less, and still further preferably 98% by mass or less, based on 100% by mass of the composition (II), from the standpoint of achieving the further excellent strength and toughness of the denture. As described above, the lower limit values and the upper limit values described in a stepwise manner can be independently combined. For example, in one embodiment of the present invention, the content of the component (B) in the composition for a prosthetic tooth (II) is preferably 40 to 100% by mass, more preferably 45 to 99.99% by mass, further preferably 50 to 99.99% by mass, still further preferably 60 to 99.5% by mass, still more further preferably 80 to 99% by mass, even further preferably 90 to 99% by mass, and even still further preferably 95 to 98% by mass, based on 100% by mass of the composition (II).

<Additional Components>

The composition for a prosthetic tooth (II) may contain a component other than the component (B) depending on necessity in such a range that does not impair the spirit of the present invention. The composition (II) can be produced according to a known method.

The composition (II) preferably contains a photopolymerization initiator from the standpoint of achieving the further excellent strength and toughness of the denture.

Examples of the photopolymerization initiator that the composition (II) can use include the same photopolymerization initiators as described for the photopolymerization initiator contained in the composition (I), and the kinds of the preferred photopolymerization initiators therefor are also the same as the description for the composition (I).

In the case where the composition (II) contains a photopolymerization initiator, the preferred ranges of the content thereof are also the same as described for the photopolymerization initiator contained in the composition (I) except that the description "composition (I)" is changed to "composition (II), and the description "per 100 parts by mass of the component (A)" is changed to "per 100 parts by mass of the component (B)".

The composition (II) may contain a colorant from the standpoint of regulating the color of the prosthetic tooth to the tooth crown color or the like. The method of regulating the color of the composition (II) is not particularly limited, and the color thereof can be regulated, for example, by mixing a known colorant. Examples of the colorant include the same colorants that have been described as the colorants that the composition (I) may contain.

In one embodiment of the present invention, the composition (II) preferably satisfies the following condition (2).

Condition (2): The chromaticity on white background according to the L*a*b* color coordinate system of a test piece having a diameter of $15.0 \pm 0.1$ mm and a thickness of $1.00 \pm 0.01$ mm formed of a cured product of the composition for a prosthetic tooth (II) is $70.0 \leq L*white \leq 90.0$, $-4.0 \leq a*white \leq 10.0$, $10.0 \leq b*white \leq 35.0$, and $10.0 \leq \Delta L \leq 40.0$.

For example, in the case where the color of the composition for a prosthetic tooth (II) is to be regulated to satisfy the range of the condition (2), it is preferred that titanium oxide or alumina are used as a major component, and the amounts of red iron oxide, yellow iron oxide, and black iron oxide added thereto are regulated depending on the desired color. The expression "titanium oxide or alumina are used as a major component" means that the colorant that has the largest content in the colorants added to the composition is titanium oxide or alumina.

In one embodiment of the present invention, the brightness difference $\Delta L$ between the brightness L*black obtained by measuring on black background and the brightness L*white obtained by measuring on white background according to the L*a*b* color coordinate system of a test piece having a diameter of $15.0 \pm 0.1$ mm and a thickness of $1.00 \pm 0.01$ mm formed of a cured product of the composition for a prosthetic tooth (II) is preferably 10.0 or more and 40.0 or less, more preferably 10.0 or more and 20.0 or less, and further preferably 10.0 or more and 18.0 or less.

In the case where the composition (II) contains the colorant, the total content of the colorant in the composition (II) is not particularly limited, and for example, is preferably 0.0005 to 5.0 parts by mass per 100 parts by mass of the component (B) from the standpoint of the color of the prosthetic tooth and the toughness of the denture. The total content of the colorant in the composition (II) is preferably 0.0005 part by mass or more since a desired color can be easily imparted thereby. The total content of the colorant in the composition (II) is preferably 5.0 parts by mass or less since the influence thereof on the curability of the composition (II) can be suppressed, and the toughness of the denture can be prevented from being decreased thereby. From this standpoint, the total content of the colorant in the composition (II) is more preferably 0.0008 part by mass or more, further preferably 0.001 part by mass or more, and still further preferably 0.005 part by mass or more, and is more preferably 1.0 part by mass or less, and further preferably 0.5 part by mass or less, per 100 parts by mass of the component (B). As described above, the lower limit values and the upper limit values described in a stepwise manner can be independently combined. For example, in one embodiment of the present invention, in the case where the composition (II) contains the colorant, the total content of the colorant in the composition (II) is preferably 0.0005 to 5.0 parts by mass, more preferably 0.0008 to 1.0 part by mass, further preferably 0.001 to 0.5 part by mass, and still further preferably 0.005 to 0.5 parts by mass, per 100 parts by mass of the component (B).

In the case where the colorant is a pigment, the preferred ranges of the total content thereof are the same as the total content of the colorant.

The composition (II) may contain a polymerization accelerator in a range that does not impair the spirit of the present invention. Examples of the polymerization accelerator that the composition (II) can use include the same photopolymerization accelerators as described for the photopolymerization accelerator that the composition (I) may contain. One kind of the polymerization accelerator may be used alone, or two or more kinds thereof may be used in combination.

The composition (II) may contain a filler for imparting the strength and the antibacterial activity to the prosthetic tooth. Examples of the filler include an organic filler, an inorganic filler, and an organic-inorganic composite filler, and also include the same fillers as described for the filler that the composition (I) may contain. One kind of the filler may be used alone, or two or more kinds thereof may be used in combination.

In one embodiment of the present invention, in the case where the composition (II) contains the filler, for example, the content of the filler in the composition (II) is preferably 150 parts by mass or less, more preferably 125 parts by mass or less, further preferably 110 parts by mass or less, and still further preferably 105 parts by mass or less, per 100 parts by mass of the component (B), from the standpoint of suppressing the influence thereof on formability due to the viscosity increase of the composition (II). In the case where the composition (II) contains the filler, the lower limit of the content of the filler is not particularly limited, and for example, is preferably 1 part by mass or more, more preferably 5 parts by mass or more, further preferably 10 parts by mass or more, still further preferably 25 parts by mass or more, and still more further preferably 40 parts by mass or more, per 100 parts by mass of the component (B), from the standpoint of imparting the strength and the antibacterial activity to the prosthetic tooth. As described above, the lower limit values and the upper limit values described in a stepwise manner can be independently combined. For example, in one embodiment of the present invention, in the case where the composition (II) contains the filler, for example, the content of the filler in the composition (II) is preferably 1 to 150 parts by mass, more preferably 5 to 150 parts by mass, further preferably 10 to 125 parts by mass, still further preferably 25 to 110 parts by mass, and still more further preferably 40 to 105 parts by mass, per 100 parts by mass of the component (B), from the standpoint of suppressing the influence thereof on formability due to the viscosity increase of the composition (II).

In one embodiment of the present invention, in the case where the composition (II) contains the filler, for example, the content of the filler in the composition (II) is preferably 20 parts by mass or less, more preferably 10 parts by mass or less, and further preferably 5 parts by mass or less, per 100 parts by mass of the component (B), from the standpoint of further suppressing the influence thereof on formability due to the viscosity increase of the composition (II) and the standpoint of achieving the further excellent strength and toughness of the denture. In the embodiment of the present invention, the content of the filler in the composition (II) may be preferably 0.1 part by mass or more, and more preferably 0.5 part by mass or more, per 100 parts by mass of the component (B). As described above, the lower limit values and the upper limit values described in a stepwise manner can be independently combined. For example, in one embodiment of the present invention, in the case where the composition (II) contains the filler, for example, the content of the filler in the composition (II) is preferably 0.1 to 20 parts by mass, more preferably 0.1 to 10 parts by mass, and further preferably 0.5 to 5 parts by mass, per 100 parts by mass of the component (B), from the standpoint of further suppressing the influence thereof on formability due to the viscosity increase of the composition (II) and the standpoint of achieving the further excellent strength and toughness of the denture.

The composition (II) may contain a polymer in such a range that does not impair the spirit of the present invention. Examples of the polymer that the composition (II) may use include the same polymers as described for the polymer that the composition (I) may contain. One kind of the polymer may be used alone, or two or more kinds thereof may be used in combination.

The composition (II) may contain a known stabilizer for the purpose of preventing the deterioration or regulating the curability. Examples of the stabilizer include a polymerization inhibitor, an ultraviolet ray absorbent, and an antioxidant.

Examples of the polymerization inhibitor that the composition (II) may use include the same polymerization inhibitors as described for the polymer that the composition (I) may contain. One kind of the polymerization inhibitor may be used alone, or two or more kinds thereof may be used in combination.

In the case where the composition (II) contains the polymerization inhibitor, the content of the polymerization inhibitor in the composition (II) is preferably 0.001 to 5.0 parts by mass, more preferably 0.01 to 3.0 parts by mass, and further preferably 0.1 to 2.0 parts by mass, per 100 parts by mass of the component (B).

The composition (II) may contain a known additional additive depending on necessity, for example, for regulating the paste properties, in addition to the components described above in such a range that does not impair the spirit of the present invention. Examples of the additional additive include a thickener, a softener, a prepolymer (oligomer), and an aryl compound (such as an aryl iodonium salt). One kind of the additional additive may be used alone, or two or more kinds thereof may be used in combination.

[Adhesive Composition]

In one embodiment of the present invention, the plate denture production kit for stereolithography is preferably an embodiment that includes an adhesive composition containing a composition (III) containing a (meth)acrylic based polymerizable monomer component (C) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (C), in addition to the curable composition for a denture base (I) and the composition for a prosthetic tooth (II).

The adhesive composition is used as an adhesive for adhering a denture base produced from the composition (I) and a prosthetic tooth produced from the composition (II).

<Composition (III)>

The composition (III) contains a (meth)acrylic based polymerizable monomer component (C) (which may be hereinafter referred simply to as a "component (C)") containing the same (meth)acrylic based polymerizable monomer (m) as contained in the curable composition for a denture base (I).

The composition (III) preferably has curability, and more preferably has photocurability.

((Meth)acrylic based Polymerizable Monomer Component (C))

The (meth)acrylic based polymerizable monomer component (C) is a component that is constituted by one kind of a (meth)acrylic based polymerizable monomer alone or two or more kinds thereof, and contains the same monomer (m) as contained in the composition (I) in an amount of 20% by mass or more based on 100% by mass of the component (C).

Accordingly, the component (C) necessarily contains the same (meth)acrylic based polymerizable monomer as the monomer (m), which is the (meth)acrylic based polymerizable monomer contained as the major monomer in the (meth)acrylic based polymerizable monomers constituting the component (A), in an amount of 20% by mass or more based on 100% by mass of the component (C).

The fact that the same (meth)acrylic based polymerizable monomer as the monomer (m) is contained in an amount of 20% by mass or more based on 100% by mass of the component (C) is the same as described for the component (B).

The adhesive composition contains the composition (III) containing the component (C) containing the particular amount of the monomer (m), which is contained in both the curable composition for a denture base (I) and the composition for a prosthetic tooth (II), and thereby is excellent in adhesion capability to both a denture base produced from the composition (I) and a prosthetic tooth produced from the composition (II).

Therefore, the embodiment in which the plate denture production kit for stereolithography further includes the adhesive composition is preferred from the standpoint of achieving the further excellent strength and toughness of a denture that is obtained by adhering the denture base and the prosthetic tooth.

As described above, the content of the monomer (m) in the component (C) is preferably 30% by mass or more, more preferably 40% by mass or more, further preferably 50% by mass or more, still further preferably 55% by mass or more, still more further preferably 60% by mass or more, and even further preferably 65% by mass or more, and is for example, 100% by mass or less, preferably 98% by mass or less, more preferably 95% by mass or less, further preferably 90% by mass or less, still further preferably 85% by mass or less, and still more further preferably 80% by mass or less, based on 100% by mass of the component (C), from the standpoint improving the adhesion the denture base and the prosthetic tooth and achieving the further excellent strength and toughness of a denture. As described above, the lower limit values and the upper limit values described in a stepwise manner can be independently combined. For example, in one embodiment of the present invention, the content of the monomer (m) in the component (C) is 20 to 100% by mass, preferably 30 to 100% by mass, more preferably 40 to 98% by mass, further preferably 50 to 95% by mass, still further preferably 55 to 90% by mass, still more further preferably 60 to 85% by mass, and even further preferably 65 to 80% by mass, based on 100% by mass of the component (C).

The (meth)acrylic based polymerizable monomer used for constituting the component (C) may be the similar (meth)acrylic based polymerizable monomer as described above for the component (A), and the preferred kinds of the (meth)acrylic based polymerizable monomer therefor are also the same as described for the component (A). The content of the (meth)acrylic based polymerizable monomers that the component (C) may contain and the preferred ranges thereof are also the same as described for the component (A) except that the description "based on 100% by mass of the component (A)" is changed to "based on 100% by mass of the component (C)", as far as satisfying the requirement to contain the monomer (m) in an amount of 20% by mass or more based on 100% by mass of the component (C).

Accordingly, these factors are also the same as described for the (meth)acrylic based polymerizable monomer constituting the component (B). Furthermore, the reasons for the preferred embodiments and the preferred ranges thereof are also the same as described above unless otherwise indicated.

As described above, the component (C) is a component that is constituted only by the monomer (m) alone, or by the monomer (m) and the monomer (o). The component (C) may contain multiple kinds of the monomer (o). Therefore, the total content of the monomer (m) and the monomer (o) in the component (C) is 100% by mass.

In one embodiment of the present invention, it is preferred that not only the component (C) contains the same monomer (m) as the component (A), but also the monomer (o) contained therein is the same (meth)acrylic based polymerizable monomer as the monomer (o) contained in the component (A). In the case where the component (A) contains multiple compounds as the monomer (o), the component (C) preferably contains all the multiple compounds as the monomer (o).

In one embodiment of the present invention, furthermore, the absolute value of the difference between the content of the monomer (m) in the component (C) and the content of the monomer (m) in the component (A) is preferably 10% by mass or less, more preferably 5% by mass or less, further preferably 3% by mass or less, and still further preferably 1% by mass or less. In other words, in one embodiment of the present invention, the absolute value of the difference between the content of the monomer (m) in the component (C) and the content of the monomer (m) in the component (A) is preferably 0 to 10% by mass, more preferably 0 to 5% by mass, further preferably 0 to 3% by mass, and still further preferably 0 to 1% by mass.

Similarly, the absolute value of the difference between the content of the monomer (o) in the component (C) and the content of the monomer (o) in the component (A) is preferably 10% by mass or less, more preferably 5% by mass or less, further preferably 3% by mass or less, and still further preferably 1% by mass or less. In other words, in one embodiment of the present invention, the absolute value of the difference between the content of the monomer (o) in the component (C) and the content of the monomer (o) in the component (A) is preferably 0 to 10% by mass, more preferably 0 to 5% by mass, further preferably 0 to 3% by mass, and still further preferably 0 to 1% by mass. In the case where the component (A) contains multiple compounds as the monomer (o), and the component (C) also contains the same multiple compounds as in the component (A) as the monomer (o), the absolute values of the differences between the content in the component (A) and the content in the component (C) of the corresponding compounds each are independently preferably 10% by mass or less, more preferably 5% by mass or less, further preferably 3% by mass or less, and still further preferably 1% by mass or less. In other words, in one embodiment of the present invention, in the case where the component (A) contains multiple compounds as the monomer (o), and the component (C) also contains the same multiple compounds as in the component (A) as the monomer (o), the absolute values of the differences between the content in the component (A) and the content in the component (C) of the corresponding compounds each are independently preferably 0 to 10% by mass, more preferably 0 to 5% by mass, further preferably 0 to 3% by mass, and still further preferably 0 to 1% by mass.

However, as described above, the total content of the monomer (m) and the monomer (o) in the component (C) is 100% by mass.

In one embodiment of the present invention, it is possible that the component (C) contains the same monomer (m) as the component (B), and the monomer (o) contained therein is the same (meth)acrylic based polymerizable monomer as the monomer (o) contained in the component (B). In the embodiment, in the case where the component (B) contains multiple compounds as the monomer (o), the component (C) preferably contains all the multiple compounds as the monomer (o).

In the embodiment of the present invention, furthermore, the absolute value of the difference between the content of the monomer (m) in the component (C) and the content of the monomer (m) in the component (B) is preferably 10% by mass or less, more preferably 5% by mass or less, further preferably 3% by mass or less, and still further preferably 1% by mass or less. In other words, in one embodiment of the present invention, the absolute value of the difference between the content of the monomer (m) in the component (C) and the content of the monomer (m) in the component (B) is preferably 0 to 10% by mass, more preferably 0 to 5% by mass, further preferably 0 to 3% by mass, and still further preferably 0 to 1% by mass.

Similarly, the absolute value of the difference between the content of the monomer (o) in the component (C) and the content of the monomer (o) in the component (B) is preferably 10% by mass or less, more preferably 5% by mass or less, further preferably 3% by mass or less, and still further preferably 1% by mass or less. In other words, in one embodiment of the present invention, the absolute value of the difference between the content of the monomer (o) in the component (C) and the content of the monomer (o) in the component (B) is preferably 0 to 10% by mass, more preferably 0 to 5% by mass, further preferably 0 to 3% by mass, and still further preferably 0 to 1% by mass. In the case where the component (B) contains multiple compounds as the monomer (o), and the component (C) also contains the same multiple compounds as in the component (B) as the monomer (o), the absolute values of the differences between the content in the component (B) and the content in the component (C) of the corresponding compounds each are independently preferably 10% by mass or less, more preferably 5% by mass or less, further preferably 3% by mass or less, and still further preferably 1% by mass or less. In other words, in one embodiment of the present invention, in the case where the component (B) contains multiple compounds as the monomer (o), and the component (C) also contains the same multiple compounds as in the component (B) as the monomer (o), the absolute values of the differences between the content in the component (B) and the content in the component (C) of the corresponding compounds each are independently preferably 0 to 10% by mass, more preferably 0 to 5% by mass, further preferably 0 to 3% by mass, and still further preferably 0 to 1% by mass.

However, as described above, the total content of the monomer (m) and the monomer (o) in the component (C) is 100% by mass.

In one embodiment of the present invention, the component (C) preferably contains the component (A) or the component (B) in an amount of 70% by mass or more, more preferably 80% by mass or more, and further preferably 90% by mass or more, and in an amount of 100% by mass or less, based on 100% by mass of the (meth)acrylic based polymerizable monomer component (C), from the standpoint of achieving the further excellent strength and toughness of the denture. In other words, in one embodiment of the present invention, the component (C) preferably contains the component (A) or the component (B) in an amount of 70 to 100% by mass, more preferably 80 to 100% by mass, and further preferably 90 to 100% by mass, based on 100% by mass of the (meth)acrylic based polymerizable monomer component (C). In the embodiment of the present invention, the component (C) still further preferably contains the component (A) or the component (B) in an amount of 100% by mass based on 100% by mass of the (meth)acrylic based polymerizable monomer component (C). In other words, it is still further preferred to use the component (A) or the component (B) as the component (C).

In one embodiment of the present invention, it is still more further preferred to use the component (A) as the component (C) from the standpoint of achieving the further excellent strength and toughness of the denture.

In one embodiment of the present invention, the composition of the component (C) is even further preferably the same as the composition of the component (A) or the component (B), and is even more further preferably the same as the composition of the component (A), from the standpoint of achieving the further excellent strength and toughness of the denture.

The content of the component (C) in the composition (III) is preferably 40% by mass or more, more preferably 45% by mass or more, further preferably 50% by mass or more, still further preferably 60% by mass or more, still more further preferably 80% by mass or more, even further preferably 90% by mass or more, and even still further preferably 95% by mass or more, and is 100% by mass or less, preferably 99.99% by mass or less, more preferably 99.5% by mass or less, further preferably 99% by mass or less, and still further preferably 98% by mass or less, based on 100% by mass of the composition (III), from the standpoint of achieving the further excellent strength and toughness of the denture. As described above, the lower limit values and the upper limit values described in a stepwise manner can be independently combined. For example, in one embodiment of the present invention, the content of the component (C) in the composition (III) is preferably 40 to 100% by mass, more preferably 45 to 99.99% by mass, further preferably 50 to 99.99% by mass, still further preferably 60 to 99.5% by mass, still more further preferably 80 to 99% by mass, even further preferably 90 to 99% by mass, and even still further preferably 95 to 98% by mass, based on 100% by mass of the composition (III).

(Additional Components that Composition (III) may contain)

The composition (III) may contain a component other than the component (C) depending on necessity in such a range that does not impair the spirit of the present invention. The composition (III) can be produced according to a known method.

The composition (III) preferably contains a photopolymerization initiator from the standpoint of achieving the further excellent strength and toughness of the denture.

Examples of the photopolymerization initiator that the composition (III) can use include the same photopolymerization initiators as described for the photopolymerization initiator contained in the composition (I), and the kinds of the preferred photopolymerization initiators therefor are also the same as the description for the composition (I).

In the case where the composition (III) contains a photopolymerization initiator, the preferred ranges of the content thereof are also the same as described for the photopolymerization initiator contained in the composition (I) except that the description "composition (I)" is changed to "composition (III), and the description "per 100 parts by mass of the component (A)" is changed to "per 100 parts by mass of the component (C)".

The composition (III) may contain a polymerization accelerator in a range that does not impair the spirit of the present invention. Examples of the polymerization accelerator that the composition (III) can use include the same polymerization accelerators as described for the polymerization accelerator that the composition (I) may contain. One kind of the polymerization accelerator may be used alone, or two or more kinds thereof may be used in combination.

The composition (III) may contain a filler in a range that does not impair the spirit of the present invention for regulating the viscosity of the composition.

Examples of the filler that the composition (III) can use include the organic fillers, the inorganic fillers, and the organic-inorganic composite fillers described for the curable composition (I) above. One kind of the filler may be used alone, or two or more kinds thereof may be used in combination.

In the case where the composition (III) contains the filler, for example, the content of the filler in the composition (III) is preferably 0.1 part by mass or more, more preferably 0.3 part by mass or more, and further preferably 0.5 part by mass or more, per 100 parts by mass of the component (C), from the standpoint of regulating the viscosity of the composition. The content of the filler in the composition (III) is preferably 10 parts by mass or less, more preferably 5 parts by mass or less, and further preferably 3 parts by mass or less, per 100 parts by mass of the component (C), from the standpoint of suppressing the influence thereof on the coatability due to the viscosity increase of the adhesive composition and the standpoint of achieving the further excellent strength and toughness of the denture. As described above, the lower limit values and the upper limit values described in a stepwise manner can be independently combined. For example, in one embodiment of the present invention, in the case where the composition (III) contains the filler, for example, the content of the filler in the composition (III) is preferably 0.1 to 10 parts by mass, more preferably 0.3 to 5 parts by mass, and further preferably 0.5 to 3 parts by mass, per 100 parts by mass of the component (C).

The composition (III) may contain a polymer in such a range that does not impair the spirit of the present invention. Examples of the polymer that the composition (III) may use include the same polymers as described for the polymer that the composition (I) may contain. One kind of the polymer may be used alone, or two or more kinds thereof may be used in combination.

The composition (III) may contain a known stabilizer for the purpose of preventing the deterioration or regulating the curability. Examples of the stabilizer include a polymerization inhibitor, an ultraviolet ray absorbent, and an antioxidant.

Examples of the polymerization inhibitor that the composition (III) may use include the same polymerization inhibitors as described for the polymer that the composition (I) may contain. One kind of the polymerization inhibitor may be used alone, or two or more kinds thereof may be used in combination.

In the case where the composition (III) contains the polymerization inhibitor, the content of the polymerization inhibitor in the composition (III) is preferably 0.001 to 5.0 parts by mass, more preferably 0.01 to 3.0 parts by mass, and further preferably 0.1 to 2.0 parts by mass, per 100 parts by mass of the component (C).

The composition (III) may contain a colorant in such a range that does not impair the spirit of the present invention, for example, for further enhancing the good appearance of the resulting denture by regulating the color of the adhesive composition to be close to the denture base or the prosthetic tooth. Examples of the colorant include the colorants described for the composition (I) above. One kind of the colorant may be used alone, or two or more kinds thereof may be used in combination.

In one embodiment of the present invention, for example, the composition (III) preferably satisfies the condition (1) or (2) above.

In the case where the color of the adhesive composition is regulated to the color of the denture base, for example, the gingival color, examples of the pigment that is mainly used include red iron oxide. In the case where the color of the adhesive composition is regulated to the color of the prosthetic tooth, for example, the tooth crown color, it is preferred that titanium oxide or alumina are used as a major component, and the amounts of red iron oxide, yellow iron oxide, and black iron oxide added thereto are regulated depending on the desired color. The expression "titanium oxide or alumina are used as a major component" means that the colorant that has the largest content in the colorants added to the composition is titanium oxide or alumina.

In the case where the composition (III) contains the colorant, the total content of the colorant in the composition (III) is not particularly limited, and for example, is preferably 0.0005 to 5.0 parts by mass per 100 parts by mass of the component (C). Therefore, in the case where the composition (III) contains the colorant, the preferred ranges of the content thereof are the same as described for the colorant contained in the composition (I) except that the description "composition (I)" is changed to "composition (III)", and the description "per 100 parts by mass of the component (A)" is changed to "per 100 parts by mass of the component (C)".

In the case where the colorant is a pigment, the preferred ranges of the total content thereof are the same as the total content of the colorant.

The composition (III) may contain a known additional additive depending on necessity, for example, for regulating the paste properties, in addition to the components described above in such a range that does not impair the spirit of the present invention. Examples of the additional additive include a thickener, a softener, a prepolymer (oligomer), and an aryl compound (such as an aryl iodonium salt). One kind of the additional additive may be used alone, or two or more kinds thereof may be used in combination.

In one embodiment of the present invention, the composition (III) preferably contains the composition (I) or the composition (II) in an amount of 70% by mass or more, more preferably 80% by mass or more, and further preferably 90% by mass or more, and is preferably 100% by mass or less, based on 100% by mass of the composition (III), from the standpoint of achieving the further excellent strength and toughness of the denture. In other words, in one embodiment of the present invention, the composition (III) preferably contains the composition (I) or the composition (II) in an amount of 70 to 100% by mass, more preferably 80 to 100% by mass, and further preferably 90 to 100% by mass, based on 100% by mass of the composition (III). In the embodiment of the present invention, the composition (III) still further preferably contains the composition (I) or the composition (II) in an amount of 100% by mass based on 100% by mass of the composition (III), and the composition (III) still more further preferably contains the composition (I) in an amount of 100% by mass based on 100% by mass of the composition (III), from the standpoint of achieving the further excellent strength and toughness of the denture. In other words, it is still further preferred to use the composition (I) or the composition (II) as the composition (III), and it is still more further preferred to use the composition (I) as the composition (III).

<Additional Components>

The adhesive composition may contain a component other than the composition (III) depending on necessity in such a range that does not impair the spirit of the present invention. For example, the composition (III) may contain a polymerizable monomer, an organic solvent, and an additional additive that are not contained in the composition (III), for regulating the coatability and the penetration of the adhesive composition to the denture base and the prosthetic tooth.

Examples of the organic solvent include ethyl acetate, butyl acetate, methylene chloride, acetone, ethanol, isopropanol, and methyl methacrylate. One kind of the organic solvents may be used alone, or two or more kinds thereof may be used in combination. Among these, ethyl acetate, ethanol, and isopropanol are preferred from the standpoint of the safety and the appropriate volatility.

In the case where the adhesive composition contains the organic solvent, the content of the organic solvent is preferably 90% by mass or less, more preferably 80% by mass or less, further preferably 60% by mass or less, still further preferably 50% by mass or less, still more further preferably 40% by mass or less, even further preferably 20% by mass or less, and even still further preferably 10% by mass or less, based on 100% by mass of the adhesive composition. In the case where the adhesive composition contains the organic solvent, the lower limit of the content of the organic solvent is not particularly limited, and is for example, preferably 1% by mass or more based on 100% by mass of the adhesive composition. As described above, the lower limit values and the upper limit values described in a stepwise manner can be independently combined. For example, in one embodiment of the present invention, in the case where the adhesive composition contains the organic solvent, the content of the organic solvent is preferably 1 to 90% by mass, more preferably 1 to 80% by mass, further preferably 1 to 60% by mass, still further preferably 1 to 50% by mass, still more further preferably 1 to 40% by mass, even further preferably 1 to 20% by mass, and even still further preferably 1 to 10% by mass, based on 100% by mass of the adhesive composition.

The content of the composition (III) is preferably 10% by mass or more, more preferably 20% by mass or more, further preferably 40% by mass or more, still further preferably 50% by mass or more, still more further preferably 60% by mass or more, even further preferably 80% by mass or more, and even still further preferably 90% by mass or more, and is preferably 100% by mass or less, based on 100% by mass of the adhesive composition, from the standpoint of achieving the further excellent strength and toughness of the denture. In other words, in one embodiment of the present invention, the content of the composition (III) is preferably 10 to 100% by mass, more preferably 20 to 100% by mass, further preferably 40 to 100% by mass, still further preferably 60 to 100% by mass, still more further preferably 80 to 100% by mass, and even further preferably 90 to 100% by mass, based on 100% by mass of the adhesive composition. In one embodiment of the present invention, the adhesive composition even still further preferably contains the composition (III) in an amount of 100% by mass based on 100% by mass of the adhesive composition. In other words, it is even still further preferred to use the composition (III) as the adhesive composition.

[Method of producing Plate Denture]

The method of producing a plate denture as one embodiment of the present invention includes adhering a denture base containing a cured product of a curable composition for a denture base (I) containing a (meth)acrylic based polymerizable monomer component (A) containing a (meth)acrylic based polymerizable monomer (m) as a major monomer, and a photopolymerization initiator, and a prosthetic tooth containing a cured product of a composition for a prosthetic tooth (II) containing a (meth)acrylic based polymerizable monomer component (B) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (B), with an adhesive composition containing a composition (III) containing a (meth)acrylic based polymerizable monomer component (C) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (C).

The curable composition for a denture base (I), the composition for a prosthetic tooth (II), the composition (III), the adhesive composition, and the components contained in the compositions are the same as those described for the plate denture production kit for stereolithography, the preferred embodiments thereof are also the same, and therefore detailed description thereof are omitted herein.

The preferred compositions of the composition (I), the composition (II), the composition (III), and the adhesive composition, i.e., the preferred amounts of the components contained in the compositions, are also the same as the contents (% by mass or parts by mass) of the components of the compositions described for the plate denture production kit for stereolithography, and therefore detailed description thereof are omitted herein.

The production method is not particularly limited, as far as including a step of adhering a denture base containing a cured product of the composition (I) and a prosthetic tooth containing a cured product of the composition (II) with the adhesive composition.

Therefore, the present invention encompasses, as one embodiment, a method of adhering a denture base and a prosthetic tooth, including adhering a denture base containing a cured product of the composition (I) and a prosthetic tooth containing a cured product of the composition (II) with the adhesive composition.

In the adhering step, the denture base and the prosthetic tooth can be adhered by coating the adhesive composition on an adhesion surface of one of the denture base and the prosthetic tooth or adhesion surfaces of both of them, bonding the denture base and the prosthetic tooth to each other via the adhesive composition, and curing the adhesive composition. In the adhering step, it is preferred that the adhesion is performed by photocuring the adhesive composition.

Specifically, for example, the production method in one embodiment preferably includes the following steps (1) and (2) in this order.

Step (1): A step of coating the adhesive composition on at least one selected from the denture base and the prosthetic tooth

Step (2): A step of adhering the denture base and the prosthetic tooth via the adhesive composition under pressure

The production method more preferably includes the following step (3) after the step (2).

Step (3): A step of adhering the denture base and the prosthetic tooth by photocuring the adhesive composition

The coating method of the adhesive composition on the denture base and/or the prosthetic tooth is not particularly limited, and known method and device or apparatus can be used. For example, the method can be manually performed with a brush or the like.

The surface of the denture base to be brought into contact with the adhesive composition may be polished in advance.

Similarly, the surface of the prosthetic tooth to be brought into contact with the adhesive composition may be polished in advance.

The method of providing the denture base containing the cured product of the composition (I) is not particularly limited, and any known method and apparatus can be used, in which a photocured product of the composition (I) is preferred, and a product obtained by stereolithography is more preferred. The stereolithography is not particularly limited, and known method and apparatus can be used. For example, a method using a CAD/CAM system or the like, such as a method using the stereolithographic 3D printer described above, can be used.

The denture base containing the cured product of the composition (I) may be a denture base containing the cured product of the composition (I) as a part thereof, or may be a denture base containing the cured product of the composition (I) as the whole thereof, and it is that at least a part of the part thereof to be brought into contact with the adhesive composition is the cured product of the composition (I), and it is preferred the whole of the part thereof to be brought into contact with the adhesive composition is the cured product of the composition (I).

The method of providing the prosthetic tooth containing the cured product of the composition (II) is also not particularly limited, and any known method and apparatus can be used, in which a photocured product of the composition (II) is preferred, and a product obtained by stereolithography is more preferred.

The prosthetic tooth containing the cured product of the composition (II) may be a prosthetic tooth containing the cured product of the composition (II) as a part thereof, or may be a prosthetic tooth containing the cured product of the composition (II) as the whole thereof, and it is preferred that at least a part of the part thereof to be brought into contact with the adhesive composition is the cured product of the composition (II), and it is more preferred that the whole of the part thereof to be brought into contact with the adhesive composition is the cured product of the composition (II).

The method of producing a plate denture as one embodiment of the present invention can produce a denture including a denture base and a prosthetic tooth united to each other through firm adhesion of the denture base and the prosthetic tooth.

In stereolithography, secondary polymerization is generally performed after producing a stereolithographic article, for the purpose of polymerizing the unreacted polymerizable monomer derived from the composition for forming the stereolithographic article. The method of performing the secondary polymerization is not particularly limited, and may be performed by using known method and apparatus, and a dental light irradiator, a secondary polymerization device for stereolithography, or the like can be used therefor.

In the case where the denture base and/or the prosthetic tooth are obtained as a stereolithographic article in the method of producing a plate denture as one embodiment of the present invention, the adhesive composition may be coated on the denture base and/or the prosthetic tooth before the secondary polymerization of the denture base and/or the prosthetic tooth, which are then adhered to each other, and thereby higher adhesiveness can be obtained.

Accordingly, in the case where the denture base and/or the prosthetic tooth are a stereolithographic article, the denture base and the prosthetic tooth each encompass a material before the secondary polymerization.

In the case where the adhesive composition is coated before the secondary polymerization, and then the secondary polymerization is performed, it is considered that the unreacted polymerizable monomer in the denture base and/or the prosthetic tooth can be copolymerized with the polymerizable monomer constituting the adhesive composition, which thus facilitate the achievement of higher adhesiveness.

The method of producing a plate denture as one embodiment of the present invention can also achieve excellent adhesiveness even in the state where the polymerization has highly progressed through the secondary polymerization and after a long period of time has passed since the stereolithography, and can produce a denture that is sufficiently excellent in adhesiveness even after a long period of time has passed since the production of the denture base and/or the prosthetic tooth.

In adhering the denture base and the prosthetic tooth by using the adhesive composition, a known dental light irradiator (for example, "α-Light V", available from J. MORITA TOKYO MFG. CORP.), a post-cure apparatus for stereolithography (for example, "Otoflash (registered trademark) G171", available from Envision TEC, Inc.), and the like can be used. The light energy used for curing the adhesive composition is preferably an active energy light ray. In other words, it is preferred that the adhesive composition is cured by irradiating the adhesive composition with an active energy light ray, so as to adhere the denture base and the prosthetic tooth.

Therefore, the step (3) is more preferably the following step (3A).

Step (3A): A step of adhering the denture base and the prosthetic tooth by curing the adhesive composition through irradiation of the adhesive composition with an active energy light ray

The "active energy light ray" herein means an energy ray that is capable of curing a resin composition for stereolithography, such as an ultraviolet ray, an electron beam, an X-ray, a radiation, and a high frequency wave. Examples of the source of the active energy light ray include an illumination, such as a halogen lamp, a xenon lamp, a metal halide lamp, an LED, a mercury lamp, and a fluorescent lamp, and a halogen lamp and an LED are preferred among these.

Examples

The present invention will be described more specifically with reference to examples below, but the present invention is not limited to the examples.

The components used in Examples and Comparative Examples and abbreviated names thereof will be described below.

[(Meth)acrylic based Polymerizable Monomer]

<Polyfunctional (Meth)acrylic based Polymerizable Monomer (a-1)>

"UDMA": 2,2,4-trimethylhexamethylenebis(2-carbamoyloxyethyl) dimethacrylate (available from Kyoeisha Chemical Co., Ltd., molecular weight: 471)
"Bis-GMA": 2,2-bis(4-(3-methacryloyloxy)-2-hydroxypropoxyphenyl)propane (available from SHIN-NAKAMURA CHEMICAL Co., Ltd.)
"TCDDMA": tricyclodecanedimethanol dimethacrylate (available from Kyoeisha Chemical Co., Ltd.)
"TEGDMA": triethylene glycol dimethacrylate (available from SHIN-NAKAMURA CHEMICAL Co., Ltd.)

<Monofunctional (Meth)acrylic based Polymerizable Monomer (a-2)>

"POBA": m-phenoxybenzyl acrylate (available from Kyoeisha Chemical Co., Ltd.)
"EPPA": ethoxylated o-phenylphenol acrylate (available from SHIN-NAKAMURA CHEMICAL Co., Ltd.)

[Photopolymerization Initiator]

"TPO": 2,4,6-trimethylbenzoyldiphenylphosphine oxide

[Polymerization Inhibitor]

"BHT": 3,5-di-t-butyl-4-hydroxytoluene

[Pigment]

"Titanium dioxide": titanium oxide, Japanese Pharmacopoeia
"Red pigment": Sicovit Red 30 E172 (available from Venator Materials PLC.)
"Yellow pigment": Sicovit Yellow 10 E172 (available from Venator Materials PLC.)
"Black pigment": Sicovit Black 80 E172 (available from Venator Materials PLC.)

[Inorganic Filler]

"Inorganic filler 1": γ-methacryloyloxypropyltrimethoxysilane-treated "Admafine (registered trademark)" SC4500-SMJ (available from Admatechs Co., Ltd., average particle diameter: 1.0 μm)

[Measurement of Chromaticity]

The chromaticities of the compositions obtained in Reference Examples were measured in the following manner.
The compositions obtained in Reference Examples each were molded into a circular disk having a diameter of 15.0 mm and a thickness of 1.0 mm with a stereolithography machine (DigitalWax (registered trade mark) 020D, available from DWS S.r.l.).
The disk was then flashed 2,000 times with a light irradiator (Otoflash (registered trademark) G171, available from Envision TEC, Inc.) to complete curing, and finished with wet sandpaper (#1000, #2000, and #3000) to have a diameter of 15.0 ± 0.1 mm and a thickness of 1.00 ± 0.01 mm, which was designated as a test piece (n = 1). The test piece in the form of a plate after polishing was measured for the L*a*b* value on white background (JIS Z8781-4:2013, colorimetry Part 4: CIE 1976 L*a*b* color space) with a spectrophotometer ("CM-3610A", available from KONICA MINOLTA, INC., D65 light source, geometric condition c (di: 8°, de: 8°), diffuse illumination: 8° light reception, SCI measurement mode, measurement diameter/illumination diameter = $\phi$ 8 mm/ $\phi$ 11 mm).
The same colorimetry was also performed on black background for measuring the L*black, and the brightness difference ΔL between white background and black background was calculated. The "white background" means the measurement of the test piece on white background with the standard white board (L* = 98.68, a* = -0.07, b* = -0.31) underneath (i.e., the side of the test piece opposite to the measurement device is allowed to be white), and the "black background" means the measurement of the test piece on white background with the standard black board (L* = 25.64, a* = -0.19, b* = -0.80) underneath (i.e., the side of the test piece opposite to the measurement device is allowed to be black).

$$\Delta L^* = L^*black - L^*white$$

Herein, L*white, a*white, and b*white mean the colorimetry results on white background, and L*black means the colorimetry result on black background.

<Reference Example 1>

[Production of Curable Composition for Denture Base (I)-1]

300 g of POBA, 30 g of TPO, 5.0 g of BHT, 0.01 g of titanium dioxide, and 0.001 g of red pigment were placed in a 2 L brown polyethylene wide mouthed bottle. After dispersing the pigment with ultrasonic wave, 700 g of UDMA was placed therein, to which a mechanical agitator was inserted, and the components were agitated at 25°C for 48 hours and confirmed complete dissolution. The resulting composition was designated as a curable composition for a denture base (I)-1. The chromaticity of a stereolithographic article (cured product) of the curable composition for a denture base (I)-1 was L*white = 78.25, a*white = 12.50, b*white = 22.74, and ΔL = 28.45.

The composition and the chromaticity of the curable composition for a denture base (I)-1 are shown in Table 1 below.

<Reference Example 2>

[Production of Curable Composition for Denture Base (I)-2]

400 g of POBA, 30 g of TPO, 5.0 g of BHT, 0.01 g of titanium dioxide, and 0.001 g of red pigment were placed in a 2 L brown polyethylene wide mouthed bottle. After dispersing the pigment with ultrasonic wave, 600 g of Bis-GMA was placed therein, to which a mechanical agitator was inserted, and the components were agitated at 25°C for 48 hours and confirmed complete dissolution. The resulting composition was designated as a curable composition for a denture base (I)-2. The chromaticity of a stereolithographic article of the curable composition for a denture base (I)-2 was L*white = 78.12, a*white = 11.93, b*white = 25.12, and ΔL = 27.88.

The composition and the chromaticity of the curable composition for a denture base (I)-2 are shown in Table 1 below.

<Reference Example 3>

[Production of Curable Composition for Denture Base (I)-3]

900 g of TCDDMA, 100 g of EPPA, 30 g of TPO, 5.0 g of BHT, 0.01 g of titanium dioxide, and 0.001 g of red pigment were placed in a 2 L brown polyethylene wide mouthed bottle. After dispersing the pigment with ultrasonic wave, a mechanical agitator was inserted thereto, and the components were agitated at 25°C for 48 hours and confirmed complete dissolution. The resulting composition was designated as a curable composition for a denture base (I)-3. The chromaticity of a stereolithographic article of the curable composition for a denture base (I)-3 was L*white = 80.03, a*white = 11.76, b*white = 19.82, and ΔL = 30.02.

The composition and the chromaticity of the curable composition for a denture base (I)-3 are shown in Table 1 below.

<Reference Example 4>

[Production of Curable Composition for Denture Base (I)-4]

300 g of TEGDMA, 30 g of TPO, 5.0 g of BHT, 0.01 g of titanium dioxide, and 0.001 g of red pigment were placed in a 2 L brown polyethylene wide mouthed bottle. After dispersing the pigment with ultrasonic wave, 700 g of UDMA was placed therein, to which a mechanical agitator was inserted, and the components were agitated at 25°C for 48 hours and confirmed complete dissolution. The resulting composition was designated as a curable composition for a denture base (I)-4. The chromaticity of a stereolithographic article of the curable composition for a denture base (I)-4 was L*white = 76.87, a*white = 14.82, b*white = 26.69, and ΔL = 25.87.

The composition and the chromaticity of the curable composition for a denture base (I)-4 are shown in Table 1 below.

<Reference Example 5>

[Production of Curable Composition for Denture Base (I)-5]

900 g of TCDDMA, 100 g of TEGDMA, 30 g of TPO, 5.0 g of BHT, 0.01 g of titanium dioxide, and 0.001 g of red pigment were placed in a 2 L brown polyethylene wide mouthed bottle. After dispersing the pigment with ultrasonic wave, a mechanical agitator was inserted thereto, and the components were agitated at 25°C for 48 hours and confirmed complete dissolution. The resulting composition was designated as a curable composition for a denture base (I)-5. The chromaticity of a stereolithographic article of the curable composition for a denture base (I)-5 was L*white = 77.13, a*white = 12.32, b*white = 21.66, and ΔL = 27.65.

The composition and the chromaticity of the curable composition for a denture base (I)-5 are shown in Table 1 below.

<Reference Example 6>

[Production of Composition for Prosthetic Tooth (II)-1]

300 g of POBA, 30 g of TPO, 5.0 g of BHT, 0.15 g of titanium dioxide, 0.002 g of red pigment, 0.005 of yellow pigment, and 0.0005 g of black pigment were placed in a 2 L brown polyethylene wide mouthed bottle. After dispersing the pigment with ultrasonic wave, 700 g of UDMA was placed therein, to which a mechanical agitator was inserted, and the components were agitated at 25°C for 48 hours and confirmed complete dissolution. The resulting composition was designated as a composition for a prosthetic tooth (II)-1. The chromaticity of a stereolithographic article of the composition for a prosthetic tooth (II)-1 was L*white = 75.75, a*white = -1.20, b*white = 18.53, and $\Delta L$ = 15.07.
The composition and the chromaticity of the curable composition for a denture base (II)-1 are shown in Table 2 below.

<Reference Example 7>

[Production of Composition for Prosthetic Tooth (II)-2]

400 g of POBA, 30 g of TPO, 5.0 g of BHT, 0.15 g of titanium dioxide, 0.002 g of red pigment, 0.005 of yellow pigment, and 0.0005 g of black pigment were placed in a 2 L brown polyethylene wide mouthed bottle. After dispersing the pigment with ultrasonic wave, 600 g of Bis-GMA was placed therein, to which a mechanical agitator was inserted, and the components were agitated at 25°C for 48 hours and confirmed complete dissolution. The resulting composition was designated as a composition for a prosthetic tooth (II)-2. The chromaticity of a stereolithographic article of the composition for a prosthetic tooth (II)-2 was L*white = 74.83, a*white = -0.63, b*white = 19.58, and $\Delta L$ = 14.48.
The composition and the chromaticity of the curable composition for a denture base (II)-2 are shown in Table 2 below.

<Reference Example 8>

[Production of Composition for Prosthetic Tooth (II)-3]

900 g of TCDDMA, 100g of EPPA, 30 g of TPO, 5.0 g of BHT, 0.15 g of titanium dioxide, 0.002 g of red pigment, 0.005 of yellow pigment, and 0.0005 g of black pigment were placed in a 2 L brown polyethylene wide mouthed bottle. After dispersing the pigment with ultrasonic wave, a mechanical agitator was inserted thereto, and the components were agitated at 25°C for 48 hours and confirmed complete dissolution. The resulting composition was designated as a composition for a prosthetic tooth (II)-3. The chromaticity of a stereolithographic article of the composition for a prosthetic tooth (II)-3 was L*white = 76.12, a*white = -1.92, b*white = 16.39, and $\Delta L$ = 16.21.
The composition and the chromaticity of the curable composition for a denture base (II)-3 are shown in Table 2 below.

<Reference Example 9>

[Production of Composition for Prosthetic Tooth (II)-4]

300 g of TEGDMA, 30 g of TPO, 5.0 g of BHT, 0.15 g of titanium dioxide, 0.002 g of red pigment, 0.005 of yellow pigment, and 0.0005 g of black pigment were placed in a 2 L brown polyethylene wide mouthed bottle. After dispersing the pigment with ultrasonic wave, 700 g of UDMA was placed therein, to which a mechanical agitator was inserted, and the components were agitated at 25°C for 48 hours and confirmed complete dissolution. The resulting composition was designated as a composition for a prosthetic tooth (II)-4. The chromaticity of a stereolithographic article of the composition for a prosthetic tooth (II)-4 was L*white = 76.11, a*white = -1.36, b*white = 17.99, and $\Delta L$ = 15.36.
The composition and the chromaticity of the curable composition for a denture base (II)-4 are shown in Table 2 below.

<Reference Example 10>

[Production of Composition for Prosthetic Tooth (II)-5]

900 g of TCDDMA, 100g of TEGDMA, 30 g of TPO, 5.0 g of BHT, 0.15 g of titanium dioxide, 0.002 g of red pigment, 0.005 of yellow pigment, and 0.0005 g of black pigment were placed in a 2 L brown polyethylene wide mouthed bottle. After dispersing the pigment with ultrasonic wave, a mechanical agitator was inserted thereto, and the components were agitated at 25°C for 48 hours and confirmed complete dissolution. The resulting composition was designated as a composition for a prosthetic tooth (II)-5. The chromaticity of a stereolithographic article of the composition for a prosthetic tooth (II)-5 was L*white = 77.02, a*white = -1.41, b*white = 17.12, and $\Delta$L = 16.21.

The composition and the chromaticity of the curable composition for a denture base (II)-5 are shown in Table 2 below.

<Reference Example 11>

[Production of Composition for Prosthetic Tooth (II)-6]

400 g of the composition for a prosthetic tooth (II)-1, 100g of TEGDMA, and 500 g of the inorganic filler 1 were placed in a 2 L brown polyethylene wide mouthed bottle. After dispersing the pigment with ultrasonic wave, a mechanical agitator was inserted thereto, and the components were agitated at 25°C for 48 hours and confirmed complete dissolution. The resulting composition was designated as a composition for a prosthetic tooth (II)-6. The chromaticity of a stereolithographic article of the composition for a prosthetic tooth (II)-6 was L*white = 73.43, a*white = 2.31, b*white = 12.43, and $\Delta$L = 13.25.

The composition and the chromaticity of the curable composition for a denture base (II)-6 are shown in Table 2 below.

<Reference Example 12>

[Production of Composition for Prosthetic Tooth (II)-7]

200 g of the composition for a prosthetic tooth (II)-1, 300g of TEGDMA, and 500 g of the inorganic filler 1 were placed in a 2 L brown polyethylene wide mouthed bottle. After dispersing the pigment with ultrasonic wave, a mechanical agitator was inserted thereto, and the components were agitated at 25°C for 48 hours and confirmed complete dissolution. The resulting composition was designated as a composition for a prosthetic tooth (II)-7. The chromaticity of a stereolithographic article of the composition for a prosthetic tooth (II)-7 was L*white = 73.58, a*white = 2.22, b*white = 12.83, and $\Delta$L = 15.25.

The composition and the chromaticity of the curable composition for a denture base (II)-7 are shown in Table 2 below.

Table 1

| | | | Unit | Reference Example 1 | Reference Example 2 | Reference Example 3 | Reference Example 4 | Reference Example 5 |
|---|---|---|---|---|---|---|---|---|
| Composition No. | | | | (I)-1 | (I)-2 | (I)-3 | (I)-4 | (I)-5 |
| Component (A) (*1) | Monomer (a-1) (*2) | UDMA | g | 700 | - | - | 700 | - |
| | | Bis-GMA | g | - | 600 | - | - | - |
| | | TCDDMA | g | - | - | 900 | - | 900 |
| | | TEGDMA | g | - | - | - | 300 | 100 |
| | Monomer (a-2) (*3) | POBA | g | 300 | 400 | - | - | - |
| | | EPPA | g | - | - | 100 | - | - |
| Additional component | Photopolymerization initiator | TPO | g | 30 | 30 | 30 | 30 | 30 |
| | Polymerization inhibitor | BHT | g | 5.0 | 5.0 | 5.0 | 5.0 | 5.0 |
| | Pigment | $TiO_2$ | g | 0.01 | 0.01 | 0.01 | 0.01 | 0.01 |
| | | Red pigment | g | 0.001 | 0.001 | 0.001 | 0.001 | 0.001 |
| Chromaticity (*4) | L*white | | - | 78.25 | 78.12 | 80.03 | 76.87 | 77.13 |
| | a*white | | - | 12.50 | 11.93 | 11.76 | 14.82 | 12.32 |
| | b*white | | - | 22.74 | 25.12 | 19.82 | 26.69 | 21.66 |
| | ΔL | | - | 28.45 | 27.88 | 30.02 | 25.87 | 27.65 |

(*1): (Meth)acrylic based polymerizable monomer component (A)
(*2): Polyfunctional (meth)acrylic based polymerizable monomer (a-1)
(*3); Monofunctional (meth)acrylic based polymerizable monomer (a-2)
(*4): Chromaticity of stereolithographic article (cured product) of composition

EP 4 403 162 A1

Table 2

| | | | Unit | Reference Example 6 | Reference Example 7 | Reference Example 8 | Reference Example 9 | Reference Example 10 | Reference Example 11 | Reference Example 12 |
|---|---|---|---|---|---|---|---|---|---|---|
| | Composition No. | | | (II)-1 | (II)-2 | (II)-3 | (II)-4 | (II)-5 | (II)-6 | (II)-7 |
| Component (B) (*5) | Monomer (a-1) (*2) | UDMA | g | 700 | - | - | 700 | - | 270 | 135 |
| | | Bis-GMA | g | - | 600 | - | - | - | - | - |
| | | TCDDMA | g | - | - | 900 | - | 900 | - | - |
| | | TEGDMA | g | - | - | - | 300 | 100 | 100 | 300 |
| | Monomer (a-2) (*3) | POBA | g | 300 | 400 | - | - | - | 116 | 58 |
| | | EPPA | g | - | - | 100 | - | - | - | - |
| Additional component | Photopolymerization initiator | TPO | g | 30 | 30 | 30 | 30 | 30 | 12 | 5.8 |
| | Polymerization inhibitor | BHT | g | 5.0 | 5.0 | 5.0 | 5.0 | 5.0 | 1.9 | 1.0 |
| | Pigment | $TiO_2$ | g | 0.15 | 0.15 | 0.15 | 0.15 | 0.15 | 0.06 | 0.03 |
| | | Red pigment | g | 0.002 | 0.002 | 0.002 | 0.002 | 0.002 | 0.001 | 0.0004 |
| | | Yellow pigment | g | 0.005 | 0.005 | 0.005 | 0.005 | 0.005 | 0.002 | 0.001 |
| | | Black pigment | g | 0.0005 | 0.0005 | 0.0005 | 0.0005 | 0.0005 | 0.0002 | 0.0001 |
| | Inorganic filler 1 | | g | - | - | - | - | - | 500 | 500 |
| Chromaticity (*4) | L*white | | - | 75.75 | 74.83 | 76.12 | 76.11 | 77.02 | 73.43 | 73.58 |
| | a*white | | - | -1.20 | -0.63 | -1.92 | -1.36 | -1.41 | 2.31 | 2.22 |
| | b*white | | - | 18.53 | 19.58 | 16.39 | 17.99 | 17.12 | 12.43 | 12.83 |
| | ΔL | | - | 15.07 | 14.48 | 16.21 | 15.36 | 16.21 | 13.25 | 15.25 |

(*2): Polyfunctional (meth)acrylic based polymerizable monomer (a-1)
(*3): Monofunctional (meth)acrylic based polymerizable monomer (a-2)
(*4): Chromaticity of stereolithographic article (cured product) of composition
(*5): (Meth)acrylic based polymerizable monomer component (B)

[Examples 1 to 7 and Comparative Examples 1 to 6]

As described below, the stereolithographic articles of the compositions described in Reference Examples were adhered according to the combinations shown in Tables 3 and 4 below with adhesives described below, and the adhesives were cured to provide test pieces, which were then subjected to the flexural strength and toughness test.

In Examples 1 to 3 and Comparative Examples 1 to 5, the compositions (III), which had the same compositions as the curable compositions for a denture base (I) used, respectively, each were used as the adhesive.

In Examples 4 and 5, the compositions (III), which had the same compositions as the compositions for a prosthetic tooth (II) used, respectively, each were used as the adhesive.

In Example 6, an adhesive composition obtained by diluting the composition (III), which had the same composition as the curable composition for a denture base (I)-1 used, with ethyl acetate to 30% by mass was used as the adhesive.

In Example 7, an adhesive composition obtained by diluting the composition (III), which had the same composition as the curable composition for a denture base (I)-1 used, with ethyl acetate to 15% by mass was used as the adhesive.

In Comparative Example 6, "UNIFAST (registered trademark) III" (available from GC Corporation, a powder-liquid type adhesive of methyl methacrylate (MMA) and polymethyl methacrylate (PMMA)) was used as the adhesive.

<Flexural Strength and Toughness Test>

The curable compositions for a denture base (I) and the compositions for a prosthetic tooth (II) obtained in Reference Examples each were subjected to stereolithography with a stereolithography machine ("DigitalWax (registered trademark) 020D", available from DWS S.r.l.), so as to produce a test piece having a length of 64.0 mm, a width of 10.0 mm, and a thickness of 1.65 mm, which was a half thickness of the dimension described in JIS T6501:2012 (Acrylic Resin for Denture Base). Subsequently, according to the combinations described in Tables 3 and 4 below, the test pieces obtained by stereolithography from the curable composition for a denture base (I) and the composition for a prosthetic tooth (II) were adhered under pressure with approximately 1 g of the adhesive described above, and after wiping off the excessive adhesive, the adhesive was flashed 2,000 times with a light irradiator ("Otoflash (registered trademark) G171", available from Envision TEC, Inc.) to complete curing, resulting in a test piece having a length of 64.0 mm, a width of 10.0 mm, and a thickness of 3.3 mm, which was the thickness of the dimension described in JIS T6501:2012 (Acrylic Resin for Denture Base). In Comparative Example 6, however, the test piece after adhering under pressure was allowed to stand in the air at 37°C for 1 hour and then similarly irradiated with the light irradiator to complete curing.

The test pieces simulating a denture according to Examples and Comparative Examples each were stored in water at 37°C for 1 day, taken out from water, and after wiping off water, subjected to a flexural strength test for evaluation, according to JIS T6501:2012. Specifically, by using a universal testing machine ("Autograph (registered trademark) AG-I 100kN", available from SHIMADZU CORPORATION), the wider surface of the test piece was placed on the two test piece supports of the jig for flexural test, so as to allow the long axis of the test piece to be perpendicular to the test piece supports and to be bilaterally symmetrical about the long axis of the apical portion of the load plunger, and the flexural strength test (n = 5) was performed at a crosshead speed of 5 mm/min. The average values of the measured values are shown in Tables 3 and 4. The flexural strength of the test piece is preferably 80 MPa or more, more preferably 90 MPa or more, and further preferably 100 MPa or more. The displacement at break thereof is preferably no breakage. As for the displacement at break, the case where breakage occurred at a displacement of 10 mm or more was evaluated as good toughness (A), the case where breakage occurred at a displacement of more than 5 mm and less than 10 mm was evaluated as moderate toughness (B), and the case where breakage occurred at a displacement of 5 mm or less was evaluated as poor toughness (F), in which the grade (B) or better was evaluated acceptable. In the case where detachment occurred at the adhesion interface in this test, there was a possibility that the flexural strength and the toughness were not correctly measured due to the adhesion force, the state of detachment was observed after the breakage.

Table 3

| | | Unit | Example 1 | Example 2 | Example 3 | Example 4 | Example 5 | Example 6 | Example 7 |
|---|---|---|---|---|---|---|---|---|---|
| Curable composition for denture base (I) | (I)-1 | % by mass | 100 | - | - | - | - | 100 | 100 |
| | (I)-2 | % by mass | - | 100 | - | - | - | - | - |
| | (I)-3 | % by mass | - | - | 100 | - | - | - | - |
| | (I)-4 | % by mass | - | - | - | 100 | - | - | - |
| | (I)-5 | % by mass | - | - | - | - | 100 | - | - |
| Composition for prosthetic tooth (II) | (II)-1 | % by mass | 100 | - | - | - | - | - | - |
| | (II)-2 | % by mass | - | 100 | - | - | - | - | - |
| | (II)-3 | % by mass | - | - | 100 | - | - | - | - |
| | (11)-4 | % by mass | - | - | - | 100 | - | - | - |
| | (II)-5 | % by mass | - | - | - | - | 100 | - | - |
| | (II)-6 | % by mass | - | - | - | - | - | 100 | - |
| | (II)-7 | % by mass | - | - | - | - | - | - | 100 |
| Adhesive composition (*6) | | | (I)-1 | (I)-2 | (I)-3 | (II)-4 | (II)-5 | (I)-1 diluted to 30% | (I)-1 diluted to 15% |
| Evaluation | Flexural strength | MPa | 115 | 110 | 98 | 92 | 88 | 93 | 87 |
| | Toughness | - | A | A | B | B | B | A | B |

(*6): The composition No. having the same composition as the adhesive composition is shown. The composition is diluted with ethyl acetate in Examples 6 and 7.

EP 4 403 162 A1

Table 4

| | | Unit | Comparative Example 1 | Comparative Example 2 | Comparative Example 3 | Comparative Example 4 | Comparative Example 5 | Comparative Example 6 |
|---|---|---|---|---|---|---|---|---|
| Curable composition for denture base (I) | (I)-1 | % by mass | 100 | - | - | - | - | 100 |
| | (I)-2 | % by mass | - | 100 | - | - | - | - |
| | (I)-3 | % by mass | - | - | 100 | - | - | - |
| | (I)-4 | % by mass | - | - | - | 100 | - | - |
| | (I)-5 | % by mass | - | - | - | - | 100 | - |
| Composition for prosthetic tooth (II) | (II)-1 | % by mass | - | 100 | - | - | - | - |
| | (II)-2 | % by mass | - | - | 100 | - | - | - |
| | (II)-3 | % by mass | - | - | - | 100 | - | - |
| | (II)-4 | % by mass | - | - | - | - | 100 | - |
| | (II)-5 | % by mass | 100 | - | - | - | - | 100 |
| Adhesive composition (*7) | | | (I)-1 | (I)-2 | (I)-3 | (I)-4 | (I)-5 | UNIFAST III |
| Evaluation | Flexural strength | MPa | 74 | 78 | 72 | 67 | 64 | 57 |
| | Toughness | - | F | B | F | F | F | F |
| (*7): The composition No. having the same composition as the adhesive composition is shown. The commercially available product was used in Comparative Example 6. | | | | | | | | |

As shown in Tables 3 and 4, the flexural strength and the toughness of the test pieces including the combination of the denture base and the prosthetic tooth produced from the curable composition for a denture base (I) and the composition for a prosthetic tooth (II) in Examples 1 to 7 are excellent compared to the strength and the toughness of the test pieces including the combination of the denture base and the prosthetic tooth produced from the curable composition for a denture base (I) and the composition for a prosthetic tooth (II) in Comparative Examples 1 to 6. All the test pieces did not suffer detachment occurring at the adhesion interface, and the flexural strength and the toughness were correctly measured.

## Claims

1. A plate denture production kit for stereolithography comprising

   a curable composition for a denture base (I) containing a (meth)acrylic based polymerizable monomer component (A) containing a (meth)acrylic based polymerizable monomer (m) as a major monomer, and a photopolymerization initiator, and
   a composition for a prosthetic tooth (II) containing a (meth)acrylic based polymerizable monomer component (B) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (B).

2. The plate denture production kit for stereolithography according to claim 1, wherein the (meth)acrylic based polymerizable monomer (m) is a polyfunctional (meth)acrylic based polymerizable monomer (a-1).

3. The plate denture production kit for stereolithography according to claim 2, wherein the polyfunctional (meth)acrylic based polymerizable monomer (a-1) is at least one kind selected from a urethanated (meth)acrylate ester compound and an aromatic compound based (meth)acrylate ester compound having no urethane bond.

4. The plate denture production kit for stereolithography according to any one of claims 1 to 3, wherein the (meth)acrylic based polymerizable monomer component (A) contains a monofunctional (meth)acrylic based polymerizable monomer (a-2).

5. The plate denture production kit for stereolithography according to any one of claims 1 to 4, wherein the (meth)acrylic based polymerizable monomer component (B) contains a monofunctional (meth)acrylic based polymerizable monomer (a-2).

6. The plate denture production kit for stereolithography according to claim 4 or 5, wherein the monofunctional (meth)acrylic based polymerizable monomer (a-2) is a (meth)acrylate ester based polymerizable monomer having an aromatic ring.

7. The plate denture production kit for stereolithography according to any one of claims 1 to 6, wherein the curable composition for a denture base (I) has a content of the (meth)acrylic based polymerizable monomer (m) of 50% by mass or more based on 100% by mass of the (meth)acrylic based polymerizable monomer component (A).

8. The plate denture production kit for stereolithography according to any one of claims 1 to 7, wherein the composition for a prosthetic tooth (II) has a content of the (meth)acrylic based polymerizable monomer (m) of 50% by mass or more based on 100% by mass of the (meth)acrylic based polymerizable monomer component (B).

9. The plate denture production kit for stereolithography according to any one of claims 1 to 8, wherein the curable composition for a denture base (I) contains the (meth)acrylic based polymerizable monomer component (A) in an amount of 40% by mass or more based on 100% by mass of the curable composition for a denture base (I).

10. The plate denture production kit for stereolithography according to any one of claims 1 to 9, wherein the composition for a prosthetic tooth (II) contains the (meth)acrylic based polymerizable monomer component (B) in an amount of 40% by mass or more based on 100% by mass of the composition for a prosthetic tooth (II).

11. The plate denture production kit for stereolithography according to any one of claims 1 to 10, wherein the plate denture production kit for stereolithography comprises

the curable composition for a denture base (I),

the composition for a prosthetic tooth (II), and

an adhesive composition containing a composition (III) containing a (meth)acrylic based polymerizable monomer component (C) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (C).

**12.** A method of producing a plate denture, comprising adhering

a denture base containing a cured product of a curable composition for a denture base (I) containing a (meth)acrylic based polymerizable monomer component (A) containing a (meth)acrylic based polymerizable monomer (m) as a major monomer, and a photopolymerization initiator, and

a prosthetic tooth containing a cured product of a composition for a prosthetic tooth (II) containing a (meth)acrylic based polymerizable monomer component (B) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (B),

with an adhesive composition containing a composition (III) containing a (meth)acrylic based polymerizable monomer component (C) containing the same (meth)acrylic based polymerizable monomer (m) as contained in the curable composition for a denture base (I), having a content of the (meth)acrylic based polymerizable monomer (m) of 20% by mass or more based on 100% by mass of the (meth)acrylic based polymerizable monomer component (C).

## INTERNATIONAL SEARCH REPORT

| International application No. |
| --- |
| **PCT/JP2022/034835** |

**A. CLASSIFICATION OF SUBJECT MATTER**

*A61K 6/887*(2020.01)i; *A61K 6/813*(2020.01)i; *A61K 6/816*(2020.01)i
FI:    A61K6/887; A61K6/816; A61K6/813

According to International Patent Classification (IPC) or to both national classification and IPC

**B. FIELDS SEARCHED**

Minimum documentation searched (classification system followed by classification symbols)

A61K6/887; A61K6/813; A61K6/816

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII), CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2016-525150 A (DENTCA, INC.) 22 August 2016 (2016-08-22)  claims, paragraphs [0016]-[0023], [0041]-[0043], [0050] | 1-3 |
| Y | | 4-12 |
| Y | JP 2019-521188 A (DENTSPLY SIRONA INC.) 25 July 2019 (2019-07-25)  claims, paragraphs [0046], [0084]-[0085], [0088]-[0090], examples 8, 9, 13, 15 | 1-12 |
| Y | WO 2018/159507 A1 (MITSUI CHEMICALS, INC.) 07 September 2018 (2018-09-07)  claims, paragraphs [0032]-[0041], [0058], [0069]-[0072], [0082]-[0088] | 1-12 |
| Y | US 2020/0383878 A1 (CYBERMED INC.) 10 December 2020 (2020-12-10)  claims, paragraph [0047], fig. 1, 2 | 1-12 |
| Y | 新井大地, 各種床用材料に対する人工歯の接着強さ -サーマルサイクルが及ぼす影響- 補綴誌, 1996, vol. 40, pages 749-758, (ARAI, Daichi. Bond Strength of Artificial Teeth to Various Denture Base Materials -Effect of Thermal Cycling-. J Jpn Prosthodont Soc.)  particularly, page 755, left column | 1-12 |
| Y | JP 2003-38527 A (GC DENTAL PRODUCTS CORP.) 12 February 2003 (2003-02-12)  paragraphs [0005]-[0006] | 1-12 |

☑ Further documents are listed in the continuation of Box C.          ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **18 November 2022** | **06 December 2022** |

| Name and mailing address of the ISA/JP | Authorized officer |
| --- | --- |
| **Japan Patent Office (ISA/JP)**  **3-4-3 Kasumigaseki, Chiyoda-ku, Tokyo 100-8915**  **Japan** |  |
|  | Telephone No. |

Form PCT/ISA/210 (second sheet) (January 2015)

**INTERNATIONAL SEARCH REPORT**

International application No.

**PCT/JP2022/034835**

**C.    DOCUMENTS CONSIDERED TO BE RELEVANT**

| Category* | Citation of document, with indication, where appropriate, of the relevant passages | Relevant to claim No. |
|---|---|---|
| Y | WO 2010/058822 A1 (NATIONAL UNIVERSITY CORPORATION TOKYO MEDICAL AND DENTAL UNIV.) 27 May 2010 (2010-05-27)<br>    paragraph [0003] | 1-12 |

Form PCT/ISA/210 (second sheet) (January 2015)

**INTERNATIONAL SEARCH REPORT**
Information on patent family members

International application No.

**PCT/JP2022/034835**

| Patent document cited in search report | | | Publication date (day/month/year) | Patent family member(s) | | Publication date (day/month/year) |
|---|---|---|---|---|---|---|
| JP | 2016-525150 | A | 22 August 2016 | WO 2014/172716 A1 claims, paragraphs [0011]-[0018], [0038]-[0041], [0049]-[0050] US 2014/0239527 A1 US 2014/0167300 A1 US 2016/0324730 A1 US 2018/0049954 A1 EP 2986654 A1 CN 105246929 A KR 10-2016-0055727 A CN 110423311 A | | |
| JP | 2019-521188 | A | 25 July 2019 | WO 2017/223084 A1 claims, pages 24-28, examples 8, 9, 13, 15 US 2017/0360534 A1 US 2019/0053883 A1 EP 3472218 A1 CA 3026166 A1 | | |
| WO | 2018/159507 | A1 | 07 September 2018 | EP 3586790 A1 claims, paragraphs [0036]-[0044], [0062], [0073]-[0076], [0086]-[0092] US 2019/0388198 A1 EP 3925570 A1 KR 10-2019-0103288 A CN 110312490 A | | |
| US | 2020/0383878 | A1 | 10 December 2020 | WO 2019/098791 A1 KR 10-2019-0056931 A | | |
| JP | 2003-38527 | A | 12 February 2003 | (Family: none) | | |
| WO | 2010/058822 | A1 | 27 May 2010 | US 2011/0236856 A1 paragraph [0003] | | |

Form PCT/ISA/210 (patent family annex) (January 2015)

**REFERENCES CITED IN THE DESCRIPTION**

**Patent documents cited in the description**

- WO 2018159507 A
- WO 2018181832 A
- JP 2000159621 A